# EUROPEAN PATENT SPECIFICATION

(11) **EP 4 234 087 B1**
(45) Date of publication and mention of the grant of the patent: **03.07.2024**
(21) Application number: 23157961.6
(22) Date of filing: 22.02.2023
(51) Int. Cl.: G01N 33/487, B01L 9/00

(54) **CARTRIDGE AND METHOD OF ANALYSING A BIOLOGICAL SAMPLE**
KARTUSCHE UND VERFAHREN ZUR ANALYSE EINER BIOLOGISCHEN PROBE
CARTOUCHE ET PROCÉDÉ D'ANALYSE D'UN ÉCHANTILLON BIOLOGIQUE

(30) Priority: 24.02.2022 SE 2250252
(43) Date of publication of application: 30.08.2023
(73) Proprietor: Sysmex Astrego AB, 756 51 Uppsala (SE); Sysmex Corporation, Kobe-shi, Hyogo 651-0073 (JP)
(72) Inventor: FREDRIKSSON, Robert, 743 45 Storvreta (SE); OLSSON, Mikael, 753 12 Uppsala (SE); ÖHMAN, Johan, 755 91 Uppsala (SE); SÖDERBERG, Lovisa, 752 39 Uppsala (SE); BALTEKIN, Özden, 741 93 Knivsta (SE)
(74) Representative: Barker Brettell Sweden AB

(56) References cited:
- WO-A1-2016/007068
- WO-A1-2017/181186

## Description

### TECHNICAL FILED

The present invention generally relates to a cartridge, and in particular to such a cartridge for a microfluidic chip. The present invention also generally relates to a method of analyzing a biological sample.

### BACKGROUND

Antibiotic susceptible testing (AST), also referred to as antibiotic sensitivity testing, is the measurement of the susceptibility of bacteria to antibiotics. Susceptibility testing results allows a clinician to select a suitable antibiotic or a mixture of antibiotics based on knowledge of the disease causing bacteria and their susceptibilities and resistances.

Initial AST methods were phenotypic methods involving exposing bacteria to antibiotics on agar plates or dilution in agar or broth. Such phenotypic methods include the disc diffusion method, also called the Kirby-Bauer method, in which bacteria are cultured on agar plate, and the bacterial growth near antibiotic-impregnated discs is observed. If the antibiotic inhibits microbial growth, a clear ring, or zone of inhibition, is seen around the disc. The bacteria are then classified as sensitive, intermediate, or resistant to an antibiotic by comparing the diameter of the zone of inhibition to defined thresholds, which correlate with minimum inhibitory concentrations (MICs). Other phenotypic methods include gradient methods, such as Etest, which use a plastic strip impregnated with different concentrations of antibiotics placed on agar and the growth medium is viewed after a period of incubation. The MIC can be identified based on the intersection of the teardrop-shaped zone of inhibition with the marking on the strip.

A major disadvantage with the above exemplified phenotypic methods is that they require bacteria culturing, typically at least overnight, in order to get a visual response. Hence, it takes comparatively long period of time before the results of the AST is available. Another disadvantage is that generally only a single antibiotic or a low number of antibiotics can be tested in a single AST test.

Another group of AST methods are genetic methods based on polymerase chain reaction (PCR), deoxyribonucleic acid (DNA) microarrays, or DNA chips. These genetic methods do not look at the phenotypic response of bacteria to antibiotics but rather analyze whether the bacteria possess genes that confer antibiotic resistance. The genetic methods have advantage over culturing-based phenotypic methods in terms of being more rapid. Disadvantages include required knowledge of resistance genes to be tested, expensive and typically require specifically trained personnel. In addition, sometimes genotypic profile of detected resistance genes does not always match the resistance profile seen with phenotypic method.

Microfluidic devices and chips have been proposed to rapidly and in parallel monitor the phenotypic response of bacteria to a set of antibiotics. AST based on microfluidics benefits from being rapid but does not suffer from the disadvantages associated with many genetic methods.

A prior art microfluidic device, denoted the "Mother Machine", is disclosed in Wang et al., Current Biology 2010, 20: 1099-1103. The Mother Machine allows for monitoring cells in many different cell channels in parallel.

Further microfluidic devices that are useful for analysis of biological samples are shown in WO 2016/007063 and WO 2016/007068. WO2017/181186A1 is also prior art.

Baltekin et al., PNAS 2017, 114(34): 9170-9175 discloses a fast antibiotic susceptible testing (AST) test, FASTest, using a microfluidic device.

US 7341841 and US 8071319 disclose a method for the detection of microorganisms in a sample. The method comprises contacting the sample with a biosensor concentration module, allowing microorganisms to grow for a first period of time and detecting growth of discrete microorganisms as an indication of the presence of the microorganisms.

There is still a need for a cartridge for such microfluidic chips that can be used for phenotyping cells in a sample, such as AST of bacteria.

### SUMMARY

It is a general objective to provide a cartridge for such microfluidic chips that can be used for phenotyping cells in a sample.

It is also a general objective to provide a method of analyzing a biological sample.

These and other objectives are met by the embodiments disclosed herein.

The invention is defined in the independent claims. Further embodiments are defined in the dependent claims.

An aspect of the invention relates to a cartridge comprising a chip chamber configured to house a microfluidic chip comprising a plurality of sets of cell channels configured to capture cells from a biological sample. The cartridge also comprises a sample chamber configured to receive the biological sample and to be in fluid connection with the plurality of sets of cell channels. The cartridge further comprises a plurality of medium reservoirs. Each medium reservoir of the plurality of medium reservoirs is configured to be in fluid connection with a respective set of cell channels of the plurality of sets of cell channels. The cartridge additionally comprises a culture medium source in fluid connection with the plurality of medium reservoirs and configured to supply a culture medium to the plurality of medium reservoirs.

Another aspect of the invention relates to a method of analyzing a biological sample. The method comprises transferring a biological sample comprising cells to a plurality of sets of cell channels in a microfluidic chip. The method also comprises transferring a culture medium to a plurality of medium reservoirs. Each medium reservoir of the plurality of medium reservoirs is configured to be in fluid connection with a respective set of cell channels of the plurality of sets of cell channels. At least one of the medium reservoirs of the plurality of medium reservoirs is preloaded with a defined amount of an agent configured to be dissolved or dispersed in the culture medium. The method further comprises transferring culture medium with the dissolved or dispersed agent from each medium reservoir, in which the agent is preloaded, to the respective set of cell channels. The method additionally comprises monitoring a response of the cells to the agent in the respective sets of cell channels.

The cartridge of the invention can be used to analyze the phenotypic response of cells in a biological sample to various agents. The cartridge is easy to use, can be pre-manufactured with all required culture medium, agents and chemicals, thereby only requiring addition of the biological sample and allows a fully automated analysis of the phenotypic response.

### BRIEF DESCRIPTION OF THE DRAWINGS

The embodiments, together with further objects and advantages thereof, may best be understood by making reference to the following description taken together with the accompanying drawings, in which:
Figs. 1A and 1B are exploded views of a cartridge according to an embodiment from above (1A) and from bottom (1B).
Figs. 2A to 2H schematically show assembly of the cartridge according to an embodiment with attachment of sample filter (2A), attachment of back lid (2B), assembly (2C) and attachment (2D) of microfluidic chip, attachment of valves and dome shaped pump (2E), attachment of medium blister (2F), addition of gas permeable membranes (2G) and attachment of top lid (2H).
Figs. 3A and 3B schematically show filling a biological sample into a sample chamber (3A) and closing the sample chamber with a cap (3B) according to an embodiment.
Figs. 4A and 4B illustrate an embodiment of the substrate from above (4A) and from bottom (4B).
Fig. 5 is a close-up view of a portion of the substrate illustrating an embodiment of the blister chamber.
Fig. 6 is a close-up view of a portion of the substrate illustrating an embodiment of the medium valve chamber.
Fig. 7 is a close-up view of a portion of the substrate illustrating an embodiment of medium reservoirs.
Fig. 8 is a close-up view of a portion of the substrate illustrating an embodiment of the sample valve chamber and surfactant chamber.
Fig. 9 is a close-up view of a portion of the substrate illustrating an embodiment of the back channel reservoir.
Fig. 10 is a close-up view of a portion of the substrate illustrating an embodiment of the dome pump chamber.
Fig. 11 is a close-up view of a portion of the substrate illustrating an embodiment of the sample reservoir.
Fig. 12 schematically illustrates the microfluidic chip attached to the chip carrier in a bottom view.
Fig. 13 schematically illustrates the microfluidic chip attached to the chip carrier in a partly transparent view.
Fig. 14 a close-up view of a portion of the microfluidic chip.
Fig. 15 is a close-up view of a portion of the blister chamber.
Fig. 16 is a close-up view (top) and cross-sectional view (bottom) of a medium reservoir.
Fig. 17 schematically illustrates the transfer of sample and culture medium within the cartridge according to an embodiment.

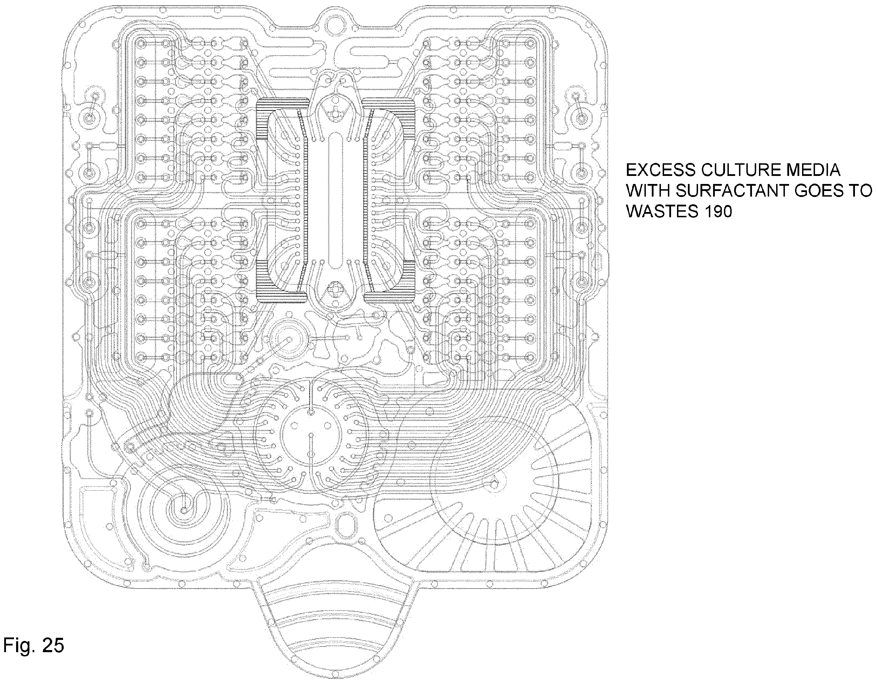

Figs. 18 to 27 schematically illustrate the cartridge during sample and culture medium filling operations as indicated in Fig. 17.
Fig. 28 schematically illustrates the microfluidic chip and fluids input to or output from entrance holes.
Figs. 29 to 31 schematically illustrate the microfluidic chip during sample and culture medium filling operations.
Fig. 32 is a flow chart illustrating a method of analyzing a biological sample according to an embodiment.

### DETAILED DESCRIPTION

Throughout the drawings, the same reference numbers are used for similar or corresponding elements.

The present invention relates to a cartridge for a microfluidic chip that can be used to analyze the phenotypic response of cells in a biological sample to various agents. The cartridge is easy to use, can be pre-manufactured with all required culture medium, agents and chemicals, thereby only requiring addition of the biological sample and allows a fully automated analysis of the phenotypic response. The present invention also relates to a method of analyzing a biological sample.

"Biological sample" as used herein include any sample, preferably fluid sample, and more preferably liquid sample, comprising cells or microorganisms, such as unicellular microorganisms, which are to be captured in a microfluidic chip of the cartridge and the phenotypic response of which to one or more agents is to be analyzed and determined. The biological sample is preferably a body fluid sample, such as a body fluid sample taken from a patient, including a processed body fluid sample taken from a patient. Non-limiting, but illustrative, examples of such body fluid samples include urine, blood, plasma, serum, amniotic fluid, cerebrospinal fluid, lymph, saliva and synovial fluid. Alternatively, the biological sample could be a biological sample obtained from a solid body sample, such as a biopsy, in which cells of the solid body sample have been suspended or dispersed in a fluid, preferably a liquid, such as a culture medium. A processed body fluid sample as used herein is a body fluid sample that has been processed in some way prior to loaded into the cartridge of the invention. For example, a body fluid sample could be filtered or separated into different sample portions to get a processed body fluid sample, such as a serum or plasma sample obtained from blood.

"Agent" as used herein relates to any molecule, compound, composition or other agent that may exert an effect on cells captured in the microfluidic chip. Typical, but non-limiting, examples of such agents include drugs or medicaments, including drug candidates. The agents do not necessarily have to be drugs or medicaments but may, for instance, be other chemicals, where there may be an interest to determine whether the agents have any effects on the cells. Illustrative, but non-limiting, examples of such agents include antimicrobial agents, such as antibacterials, antifungals, antivirals, or antiparasitics. Particular examples include antibacterials, i.e., antibiotics, including, but not-limited to beta-lactams, cephalosporins, sulfonamides, aminoglycosides, chloramphenicol, tetracyclines, macrolides, glycopeptides, ansamycins, quinolones, streptogramins, oxazolidinones, and lipopeptides.

"Cell" as used herein includes any cell type or population or mixture of cells or cell populations present in a biological sample and that can be captured in the microfluidic chip. The cell could, for instance, be a pathogen that may cause a disease or disorder in a subject. Non-limiting, but illustrative, examples of such pathogens include bacteria, algae, fungi, protozoans and other parasites. The cell may alternatively be a pathogen host for the pathogen, such as for viroids, viruses, or prions. The cell does not necessarily have to be a pathogen but could alternatively be any cell type or cell population to be captured and monitored, including microorganisms, and in particular unicellular microorganisms.

In an illustrative example, the cartridge could be used in AST of bacteria in a biological sample, in particular a urine sample taken from a subject suffering from urine tract infection (UTI), and with antibiotics as examples of agents. The cartridge of the invention is, however, not limited to usage with urine sample as biological sample. In fact, the cartridge could be used to load bacteria, or indeed any type of cell or microorganisms, into a microfluidic chip for analysis and can thereby be used together with other biological samples.

An aspect of the invention relates to a cartridge 1 comprising a chip chamber 105 configured to house a microfluidic chip 500 comprising a plurality of sets 531 of cell channels 530 configured to capture cells from a biological sample. The cartridge 1 also comprises a sample chamber 140 configured to receive the biological sample and be in fluid connection with the plurality of sets 531 of cell channels 530. The cartridge 1 comprises a plurality of medium reservoirs 170. Each medium reservoir 170 of the plurality of medium reservoirs 170 is configured to be in fluid connection with a respective set 531 of cell channels 530 of the plurality of sets 531 of cell channels 530. The cartridge 1 additionally comprises a culture medium source 110, 400 in fluid connection with the plurality of medium reservoirs 170 and configured to supply a culture medium to the plurality of medium reservoirs 170.

The cartridge 1 of the present invention thereby enables capturing cells from a biological sample in a plurality of sets 531 of cell channels 530 in the microfluidic chip 500 housed in the chip chamber 105. The cells can then be manipulated, such as exposed to an agent at various concentrations and/or to multiple different agents, in parallel while monitoring the response to the cells to the manipulation, such as the response of the cells to the different concentrations of the agent and/or to the different agents. Hence, a single source of cells, i.e., the biological sample, can be applied to the cartridge 1 to distribute the cells provided therein into the different sets 531 of cell channels 530.

The cartridge 1 also comprises a plurality of medium reservoirs 170 so that each set 531 of cell channels 530 is in fluid connection with a respective medium reservoir 170. Hence, if the microfluidic chip 500 comprises N sets of the cell channels 530, the cartridge 1 comprises N medium reservoirs 170. This means that each set 531 of cell channels 530 is in fluid connection with its own medium reservoir 170 and is supplied with culture medium therefrom. In other words, each medium reservoir 170 of the plurality of medium reservoirs 170 only supplies culture medium to a single set 531 of cell channels 530 in the microfluidic chip 500, i.e., is only in fluid connection with a single set 531 of cell channels 530. This arrangement of medium reservoirs 170 relative to the set 531 of cell channels 530 in the microfluidic chip 500 prevents mixing of culture medium from different medium reservoirs 170 in the microfluidic chip 500 and thereby prevents exposing cells captured in a set 531 of cell channels 530 to culture medium from different medium reservoirs 170.

The cartridge 1 further comprises a culture medium source 110, 400 in fluid connection with the plurality of medium reservoirs 170. This culture medium source 110, 400 supplies culture medium to the plurality of medium reservoirs 170. Hence, in a preferred embodiment, culture medium is supplied from the culture medium source 110, 400 and distributed into the plurality of culture reservoirs 170.

In an embodiment, the plurality of medium reservoirs 170 is arranged symmetrically with respect to the chip chamber 105.

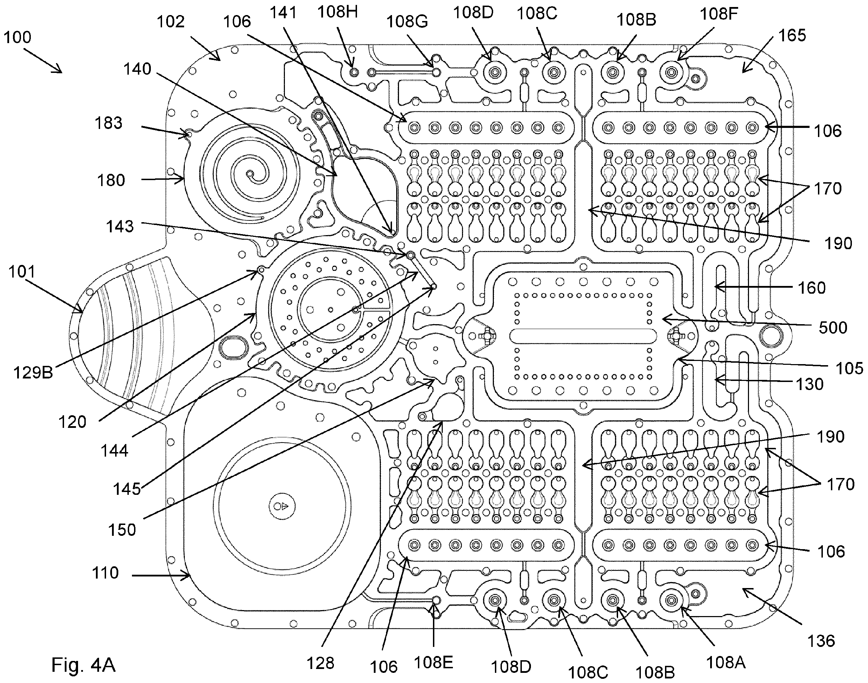

As is more clearly shown in Fig. 4A, the chip chamber 105 is preferably provided in between the plurality of medium reservoirs 170. Preferably, half of the medium reservoirs 170 are arranged on side of the chip chamber 105 with the remaining half of the medium reservoirs 170 arranged on the other side of the chip chamber 105. Hence, the chip chamber 105 is preferably arranged in between two sets or rows of medium reservoirs 170. This distribution of the medium reservoirs 170 relative to the central chip chamber 105 enables arrangement of a plurality of medium reservoirs 170 at a comparatively small space in the cartridge 1.

In an embodiment, the plurality of medium reservoirs 170 has a substantially same shape and/or internal volume.

In a currently preferred embodiment, the plurality of medium reservoirs 170 has substantially the same internal volume and preferably substantially the same shape. In such a case, each medium reservoir 170 of the plurality of medium reservoirs 170 is preferably filled with substantially the same volume of culture medium from the culture medium source 110, 400. Such a design of the medium reservoirs 170 enables achieving an accurate and controlled concentration of any agent dissolved or dispersed in the culture medium in a medium reservoir 170. For instance, a medium reservoir 170 can be preloaded with a defined amount of an agent. Filling the medium reservoir 170 with a defined volume of culture medium supplied from the culture medium source 110, 400 thereby enables formation of a well-defined concentration of the agent dissolved or dispersed in the culture medium in the medium reservoir 170. Accurate and well-defined concentrations of one or more agents can thereby be achieved in different medium reservoirs 170 if the different medium reservoirs 170 are preloaded with different amounts of the agent or agents and if the medium reservoirs 170 have substantially the same internal volume.

In an embodiment, at least some of the plurality of medium reservoirs 170 is preloaded with different amounts of an agent or different agents configured to be dissolved or dispersed in the culture medium.

As mentioned above, one or more agents preloaded in the medium reservoirs 170 will be dissolved or dispersed in the culture medium supplied to the medium reservoirs 170 from the culture medium source 110, 400. The culture medium with dissolved or dispersed agent is then supplied from a medium reservoir 170 to its connected set 531 of cell channels 530 in the microfluidic chip 500 to thereby expose cells captured in the cell channels 530 of the set 531 of cell channels 530 to the agent dissolved or dispersed in the culture medium. The response of the cells in the cell channels 530 to the agent can then be monitored and determined.

It is possible to preload multiple medium reservoirs 170 with different amounts of the same agent to thereby provide the agent at different concentrations in the culture medium contained in the medium reservoirs 170. Cells captured in different sets 531 of cell channels 530 will thereby be exposed to different concentrations of the agent and the response of the cells to these different concentrations can be monitored.

Alternatively, or in addition, different agents can be preloaded in different medium reservoirs 170 to thereby enable monitoring the response of captured cells to these different agents.

In a particular embodiment, a first set of the plurality of medium reservoirs 170 is preloaded with different amounts of the agent or different agents, and a second set of the plurality of medium reservoirs 170 is not preloaded with any agent. In a preferred embodiment, the first set of the plurality of medium reservoirs 170 is preloaded with different amounts of the agent or different agents, and the remaining set of the plurality of medium reservoirs 170 is not preloaded with any agent.

For instance, in a cartridge 1 having 32 different medium reservoirs 170, five different agents at five different concentrations could be preloaded into 25 of the 32 medium reservoirs 170 with the remaining seven medium reservoirs 170 used as various controls, such as not preloaded with any agents or preloaded with one or more control chemicals or agents.

The medium reservoir(s) 170 lacking any agent will thereby only contain culture medium. Such medium reservoir(s) 170 can be used as control for enabling monitoring the response of cells captured in connected set(s) of cell channels 530 to the culture medium. In such a case, the response of cells to one or more agents could be determined in relation to the response of cells to the culture medium.

In an embodiment, the agent is an antimicrobial agent. A currently preferred example of antimicrobial agent is antibiotics.

In an embodiment, each medium reservoir 170 of the plurality of medium reservoirs 170 comprises two separate and interconnected chambers 170A, 170B.

In such an embodiment, the culture medium supplied from the culture medium source 110, 400 is preferably supplied to a respective first or back chamber 170A of the medium reservoirs 170. The culture medium are then pushed from the first or back chambers 170A to a respective second or front chamber 170B of the medium reservoirs 170. Such an approach enables metering of a well-defined volume of the culture medium in the first or back chamber 170A and then suppling this volume of culture medium into the second or front chamber 170B. In such an embodiment, any agent preloaded in the medium reservoir 170 is preferably preloaded in the second or front chamber 170B. In case a comparatively large amount of agent need to be preloaded into the medium reservoir 170 and/or an agent needs a comparatively long period of time to become dissolved or dispersed in the culture medium, a main part of the agent is preferably preloaded in the second or front chamber 170B with a complementary amount of the agent preloaded in the first or back chamber 170A.

In an embodiment, the cartridge 1 further comprises a medium valve chamber 120. The medium valve chamber 120 comprises an entrance hole 121 in fluid communication with the culture medium source 110, 400. The medium valve chamber 120 also comprises a plurality of medium reservoir holes 123. Each medium reservoir hole 123 of the plurality of medium reservoir holes 123 is in fluid communication with a respective medium reservoir 170 of the plurality of medium reservoirs 170. The cartridge 1 also comprises a culture medium valve 420 configured to direct culture medium supplied through the entrance hole 121 from the culture medium source 110, 400 into the plurality of medium reservoir holes 123.

In a particular embodiment, the culture medium valve 420 is configured to direct culture medium supplied through the entrance hole 121 from the culture medium source 110, 400 into the plurality of medium reservoirs 170 through the plurality of medium reservoir holes 123.

The medium chamber 120 and the culture medium valve 420 are configured to redirect an inflow of culture medium from the culture medium source 110, 400 through the entrance hole 121 into a plurality of outflows of culture medium to the plurality of medium reservoirs 170 through the plurality of medium reservoir holes 123.

In an embodiment, the culture medium valve 420 is configured to block the plurality of medium reservoir holes 123 once the culture medium has been supplied to the plurality of medium reservoirs 170. The culture medium valve 420 thereby prevents or restricts any backflow of culture medium from one medium reservoir 170 into the medium chamber 120 and further into another medium reservoir 170 of the multiple medium reservoirs 170.

In an embodiment, the cartridge 1 comprises a pump 410 configured to move biological sample from the sample chamber 140 into the plurality of sets 531 of cell channels 530.

In an embodiment, the cartridge 1 comprises a surfactant chamber 128 configured to be in fluid connection or communication with the plurality of sets 531 of cell channels 530 and the culture medium source 110, 400. In such an embodiment, the surfactant chamber 128 is preloaded with a surfactant configured to be dissolved or dispersed in culture medium supplied from the culture medium source 110, 400. The surfactant dissolved or dispersed in the culture medium is configured to be supplied to the plurality of sets 531 of cell channels 530.

In a particular embodiment, the surfactant chamber 128 is preloaded with the surfactant configured to be dissolved or dispersed in culture medium supplied from the culture medium source 110, 400. The surfactant dissolved or dispersed in the culture medium is configured to be supplied to the plurality of sets 531 of cell channels 530 prior to the biological sample.

In such an embodiment, the cell channels 530 and any fluid channels of the microfluidic chip 500 are preferably wetted with the culture medium supplied with surfactant prior to loading the biological sample into the microfluidic chip 500. The surfactant preferably coats internal surfaces of cell channels 530 and any other fluid channels of the microfluidic chip 500 to thereby facilitate loading the biological sample into the microfluidic chip 500. Such a wetting thereby reduces any pressure needed to push the biological sample into the microfluidic chip 500.

In an embodiment, the culture medium source 110, 400 comprises a culture medium container 400 prepackaged with the culture medium. The culture medium source 100, 400 also comprises a chamber 110 configured to house the culture medium container 400. The chamber 110 comprises a drainage hole 113 in fluid connection or communication with the plurality of medium reservoirs 170. The chamber 110 also comprises a cutter 114 configured to cut a surface of the culture medium container 400 to supply the culture medium into the drainage hole 113.

In a particular embodiment, the culture medium container 400 is pushed by the application of a fluidic pressure, such as a gas pressure and preferably an air pressure, onto the culture medium container 400 to press the culture medium container 400 onto the cutter 114 to thereby cut a surface of the culture medium container 400.

In an embodiment, the cartridge 1 comprises a chip carrier 550 attached to the microfluidic chip 500. The chip carrier 550 comprises a first set of holes 554 configured to provide fluidic connection between the plurality of sets 531 of cell channels 530 and the sample chamber 140. The chip carrier 550 also comprises a second set of holes 558. Each hole 558 of the second set of holes 558 is configured to provide fluidic connection between a respective set 531 of cell channels 530 of the plurality of sets 531 of cell channels 530 and a respective medium reservoir 170 of the plurality of medium reservoirs 170.

In a particular embodiment, each set 531 of cell channels 530 of the plurality of sets 531 of cell channels 530 comprises a first port 514 in fluid communication with a hole 554 of the first set of holes 554 and a second port 512 in fluid communication with a hole 558 of the second set of holes 558.

In an embodiment, the cartridge 1 further comprises a substrate 100 comprising the chip chamber 105, the sample chamber 140, the plurality of medium reservoirs 170 and the culture medium source 110, 400. The cartridge 1 also comprises a lid 200 attached to the substrate 100 and comprising a window 210 configured to enable imaging the plurality of sets 531 of cell channels 530.

In an embodiment, each set 531 of cell channels 530 of the plurality of sets 531 of cell channels 530 is formed as a compartment in the microfluidic chip 500, which is separate from other sets 531 of cell channels 530 of the plurality of sets 531 of cell channels 530.

The cartridge and its included parts will now be described in more detail in connection with the accompanying drawings followed by a description a typical use of the cartridge.

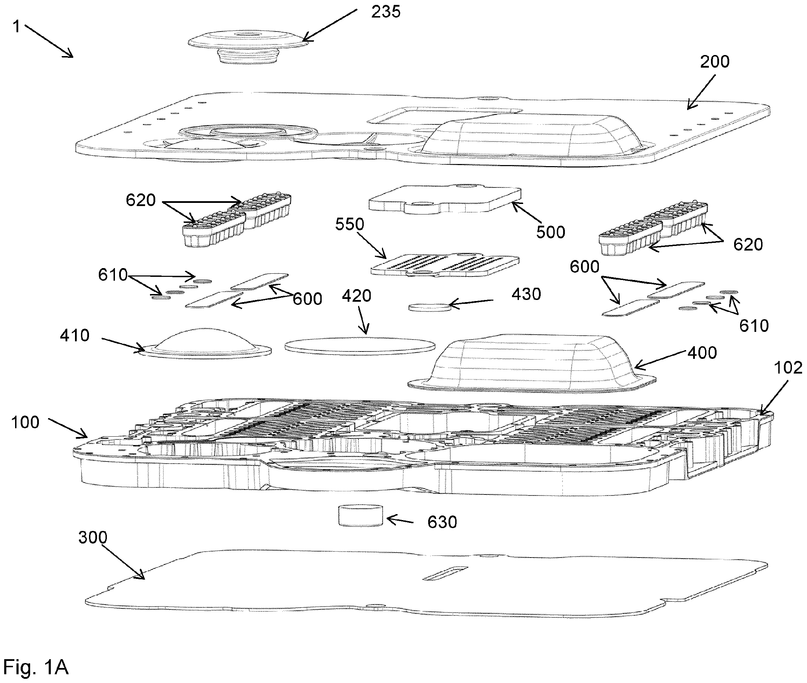

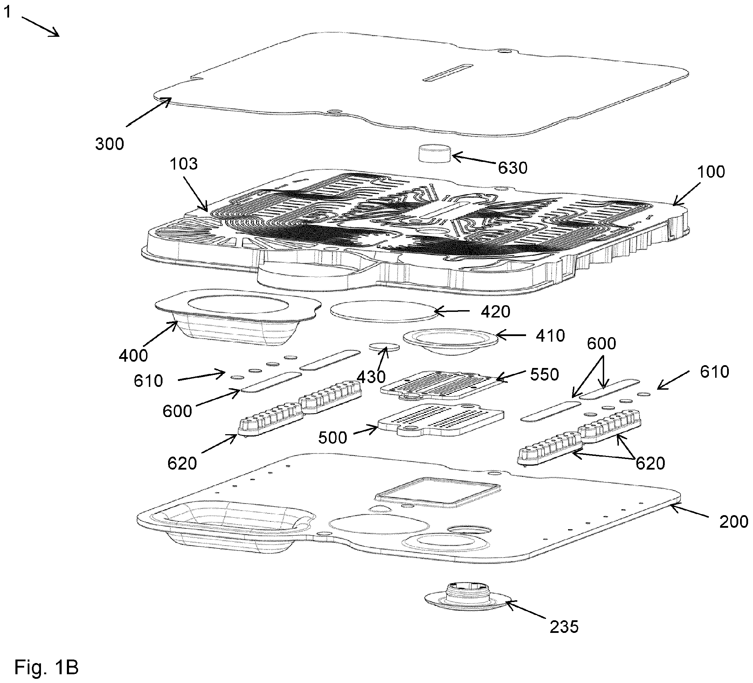

Figs. 1A and 1B are exploded views of an embodiment of a cartridge 1 according to an embodiment from above (Fig. 1A) and from bottom (Fig. 1B). The cartridge 1 comprises a substrate 100, also referred to as cartridge substrate, designed to house a microfluidic chip 500 typically attached or bonded to chip carrier 550 and a medium blister 400 comprising culture medium for the cells to be captured in the microfluidic chip 500. The cartridge 1 also comprises valves 420, 430 acting as check valves for the culture medium and the biological sample, respectively, and a dome shaped pump 410 for accurate metering of a volume of the biological sample. A sample filter 630 is preferably included in the cartridge 1 for filtering the biological sample prior to loading the biological sample into the microfluidic chip 500. The cartridge 1 further comprises a number of gas permeable membranes 600, 610 permeable to gas, such as air, but not permeable to liquid at the operating pressures. The cartridge 1 optionally comprises membrane compressors 620 to be aligned with the gas permeable membranes 600 and keep the gas permeable membranes 600 in sealed connection with the substrate 100. A top lid 200 is designed for attachment to the upper or top surface 102 of substrate 100 with a corresponding back lid 300 designed for attachment to the lower or bottom surface 103 of the substrate 100. A cap 235 is designed to fit into the top lid 200 to close a sample chamber in the substrate 100 once the biological sample has been added to a sample chamber.

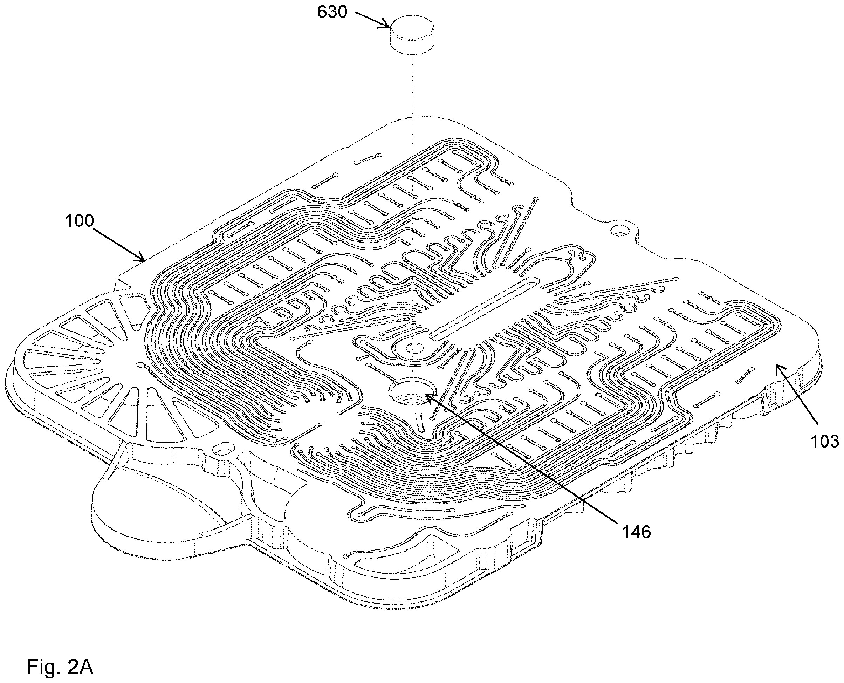

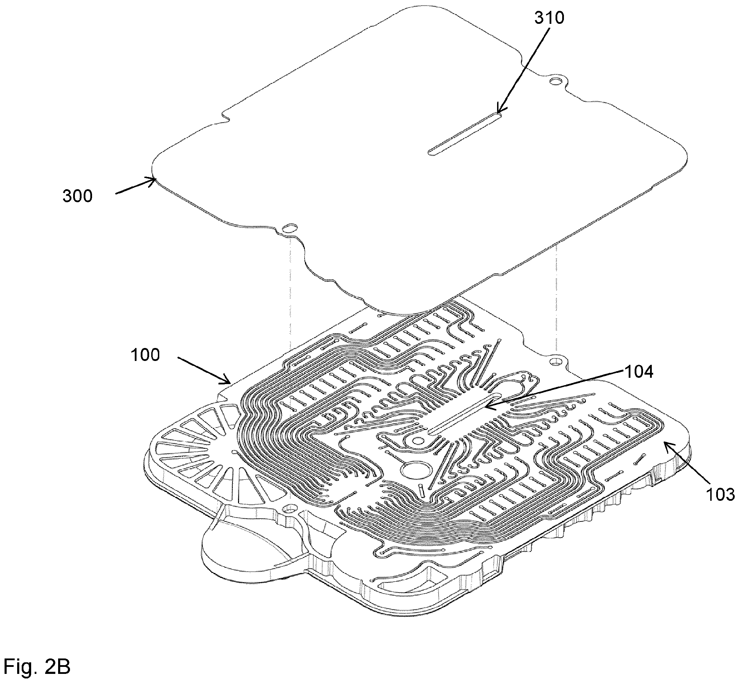

The cartridge 1 can be assembled according to various embodiments. In an illustrative assembly embodiment, the sample filter 630 is inserted and preferably press fitted into a filter chamber or well 146 having a matching opening in the bottom surface 103 of the substrate 100 as shown in Fig. 2A. The back lid 300 is then attached to the bottom surface 103 of the substrate 100, for instance by welding, such as laser welding, the back lid 300 to the substrate 100 as shown in Fig. 2B. Optional guide pins on fixture could be used to align the back lid 300 and the substrate 100 as indicated in Fig. 2B. The figure also shows that the back lid 300 comprises an opening or window 310 that is configured to be aligned with a corresponding opening 104 in the substrate 100. These openings 104, 310 are in turn, during use, configured to be aligned with a portion of the microfluidic chip 500 comprising cell channels 530, in which cells present in the biological sample are to be captured and cultured. Hence the openings 104, 310 in the substrate 100 and in the back lid 300, respectively, enables visual access to a portion of the microfluidic chip 500 as positioned in the chip chamber 105, and in particular visual access to the sets 531 of cell channels 530 in the microfluidic chip 500.

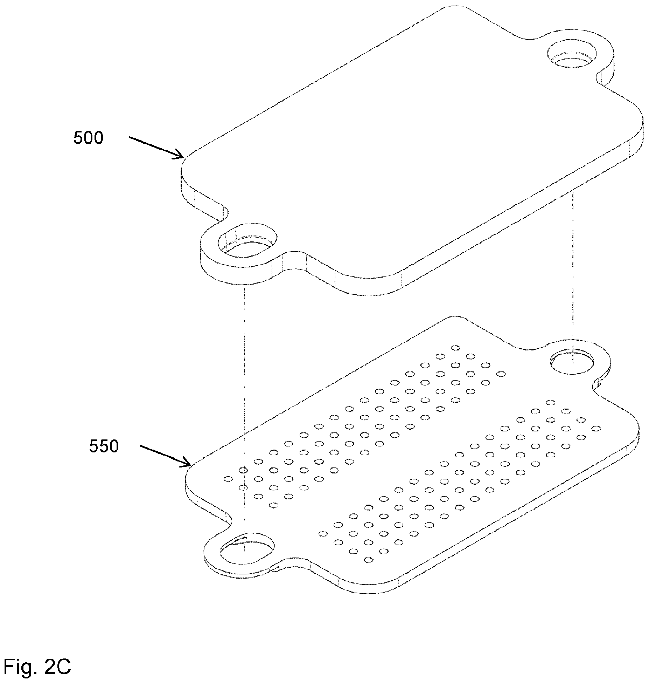

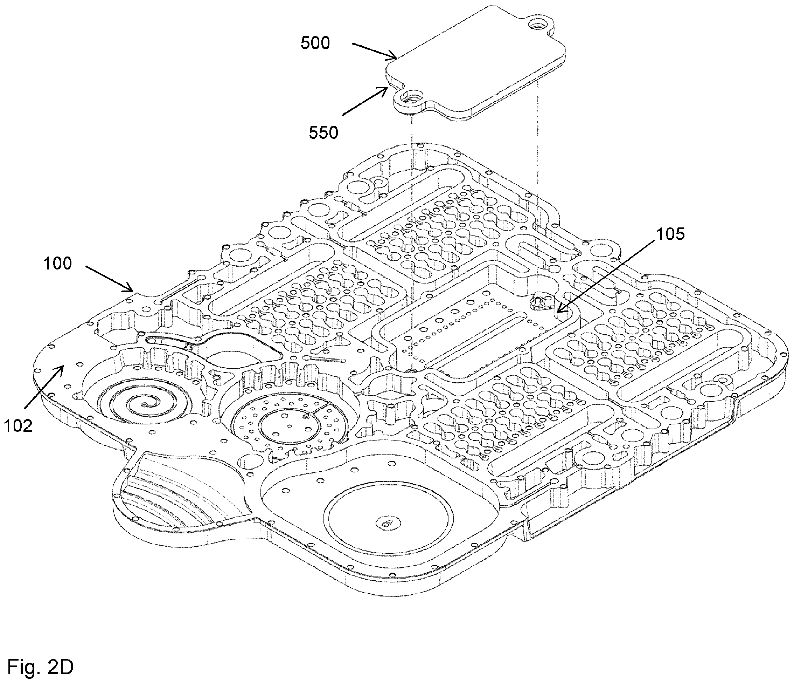

The microfluidic chip 500 is attached to the chip carrier 550, for instance by bonding the microfluidic chip 500 onto the chip carrier 550 as shown in Fig. 2C. Optional guide pins on fixture could be used to align the microfluidic chip 500 and the chip carrier 550 as indicated in Fig. 2C. The chip carrier 550 with the attached microfluidic chip 500 is then inserted into a matching chip chamber or well 105 in the substrate 100 as indicated in Fig. 2D. The chip carrier 550 is preferably attached to the substrate 100, such as by welding, preferably laser welding, the chip carrier 550 to the substrate 100. Optional guide pins on fixture could be used to align the chip carrier 550 and the substrate 100 as shown in Fig. 2D.

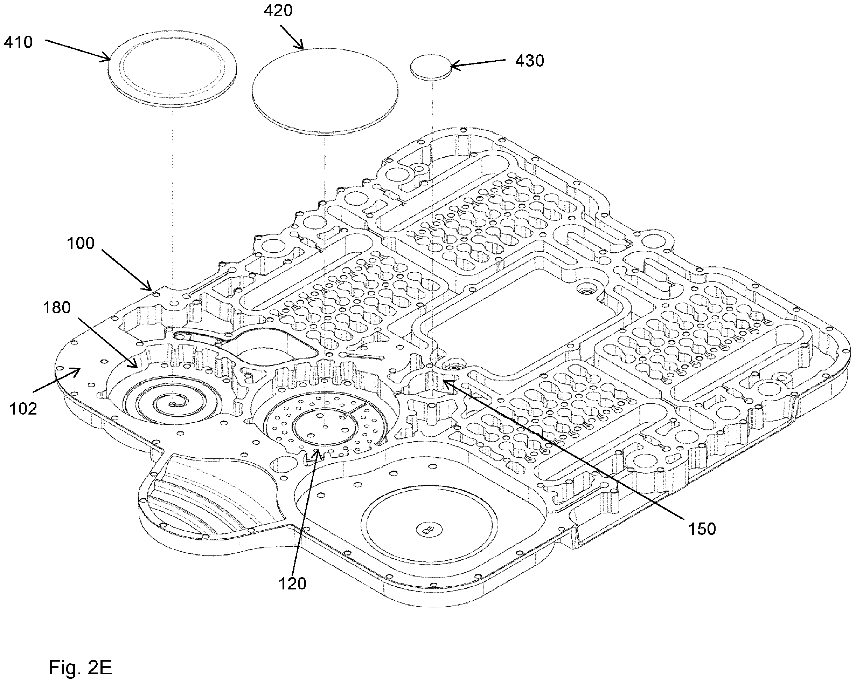

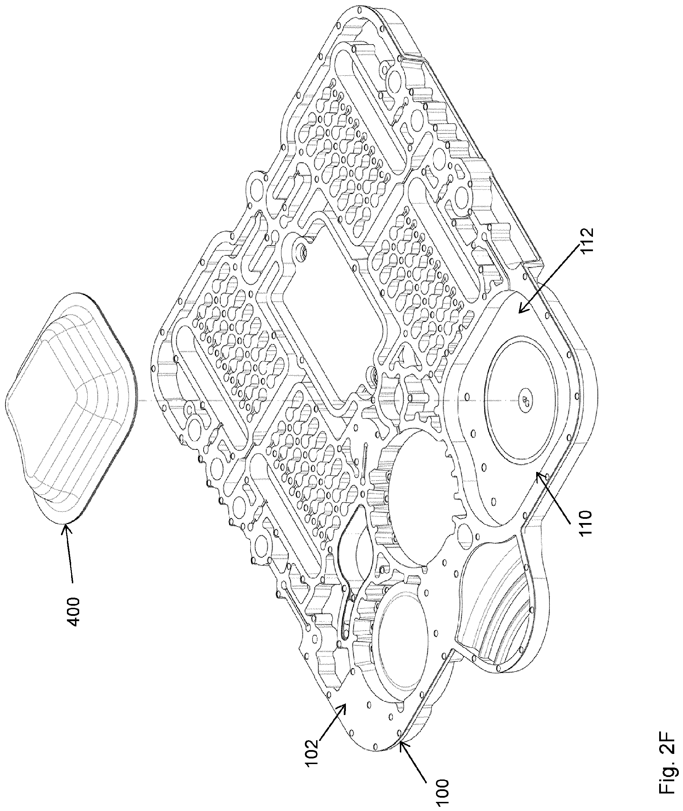

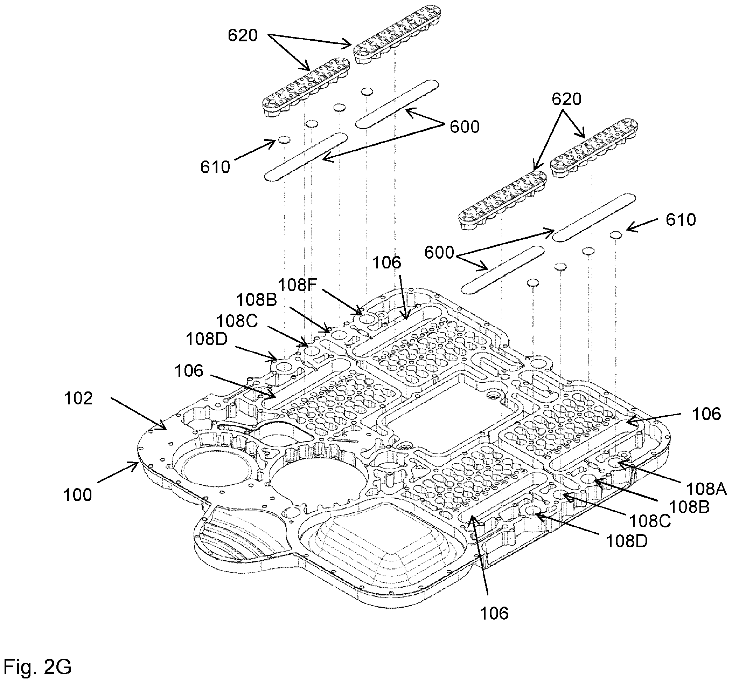

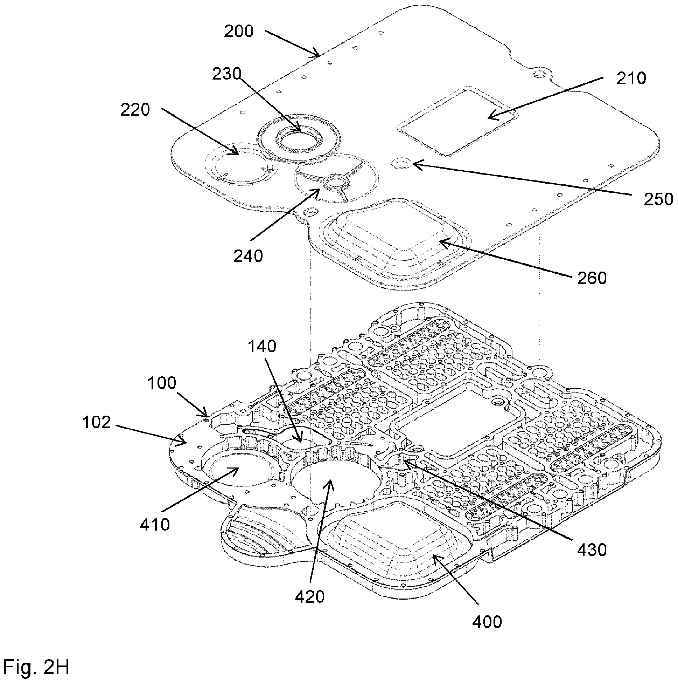

The dome shaped pump 410, culture medium valve 420 and the sample valve 430 are then inserted into respective a respective chamber or well 120, 150, 180 in the substrate 100 as shown in Fig. 2E. The dome shaped pump 410 and the valves 420, 430 are preferably welded, such as laser welded, to the substrate 100, such as by applying a weld, preferably a laser weld, along a respective circumference of the dome shaped pump 410 and the valves 420, 430 and the bottom surface of the chambers or wells 120, 150, 180. Fig. 2F schematically shows inserting the medium blister 400 into a blister chamber or well 110 in the substrate 100. The medium blister 400 is preferably attached to the substrate 100 along the circumference of the bottom surface of the medium blister 400, such as by an adhesive, for instance an adhesive tape, applied to at least a portion of the bottom surface of the medium blister 400 and/or to at least a portion of bottom surface 112 of the blister chamber or well 110. The gas permeable membranes 600 are then positioned in respective membrane chambers or wells 106 in the substrate 100 as shown in Fig. 2G. In an optional embodiment, membrane compressors 620 could be used to sealingly attach the gas permeable membranes 600 into the membrane chambers 106. Other techniques for securing the gas permeable membranes 600 in the membrane chambers 106 could be used instead, such as welding or gluing. The gas permeable membranes 610 are correspondingly inserted into respective pressure ports 108A to 108E in the substrate 100. Finally, the top lid 200 is attached to the upper surface 102 of the substrate 100 as shown in Fig. 2H, such as by welding, preferably laser welding, the top lid 200 to the substrate 100. Optional guide pins on fixture could be used to align the top lid 200 and the substrate 100 as indicated in Fig. 2H. The figure also schematically shows that the top lid 200 comprises a number of openings or windows 210, 220, 240, 250 that are configured to be aligned with the microfluidic chip 500, the dome shaped pump 410, the culture medium valve 420 and the sample valve 430, respectively. The top lid 200 preferably also comprises a raised portion acting as a blister cover 260 for the medium blister 400.

The order at which the different components of the cartridge 1 are assembled can differ from the order discussed above and shown in Figs. 2A to 2H.

The cartridge 1 of the present invention can easily be handled by a user since all components of the cartridge 1 can be assembled during manufacture and then provided as a cartridge 1 with the substrate 100 and the components enclosed by the top lid 200 and the back lid 300.

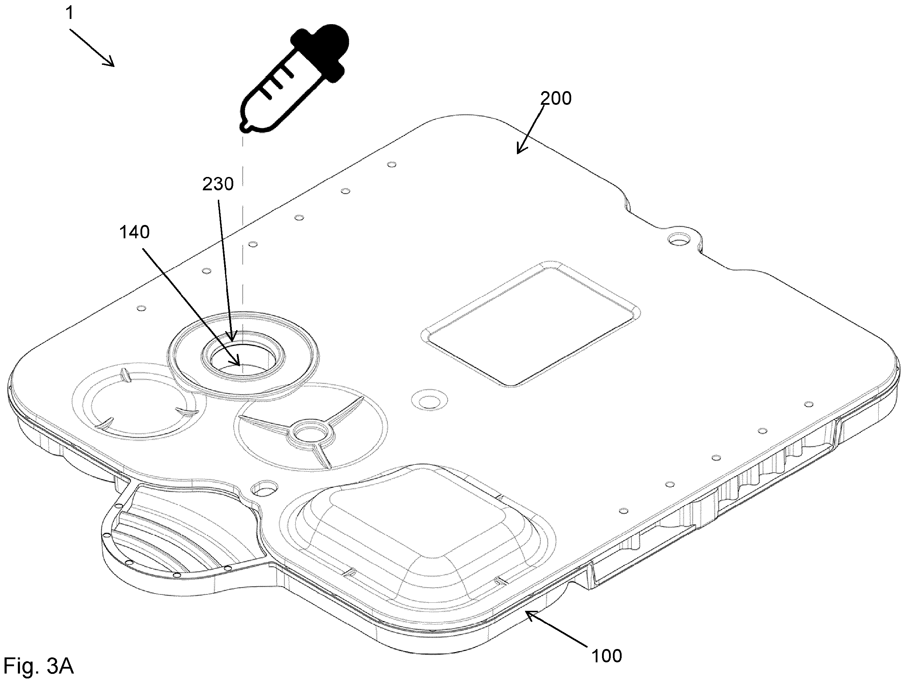

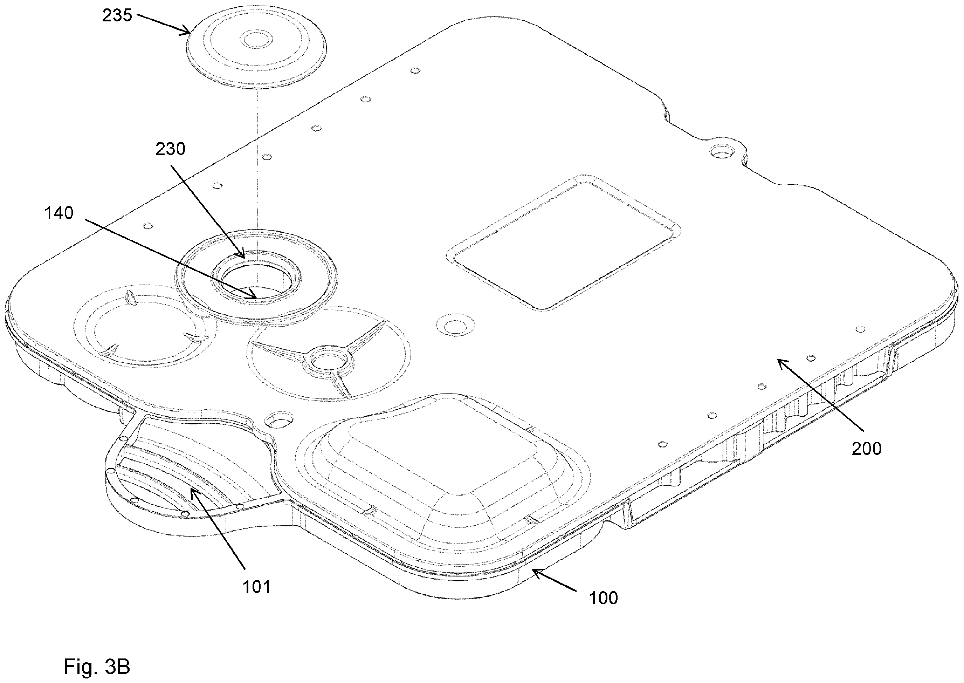

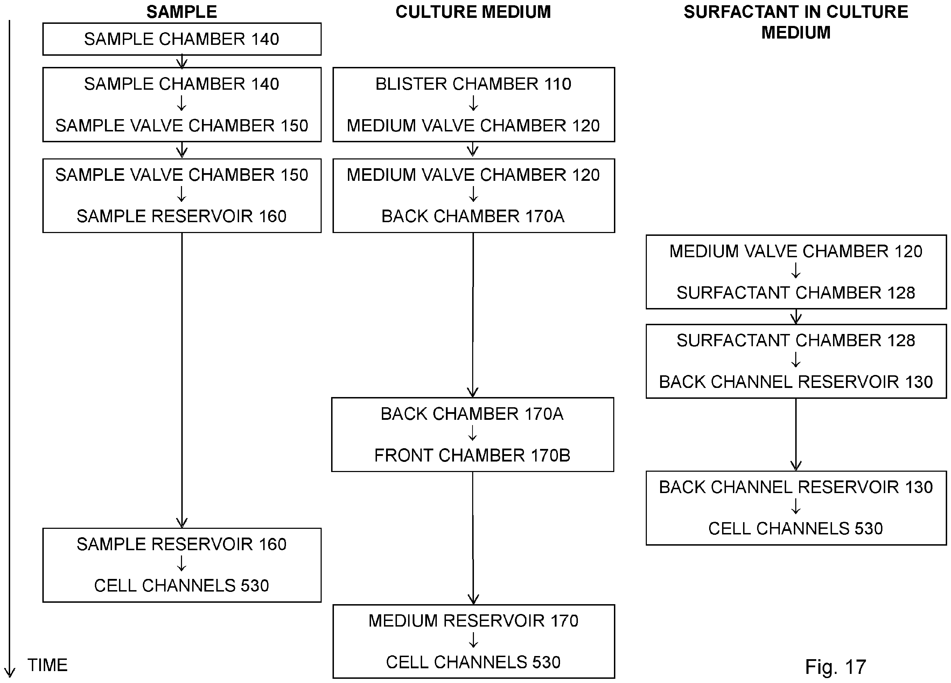

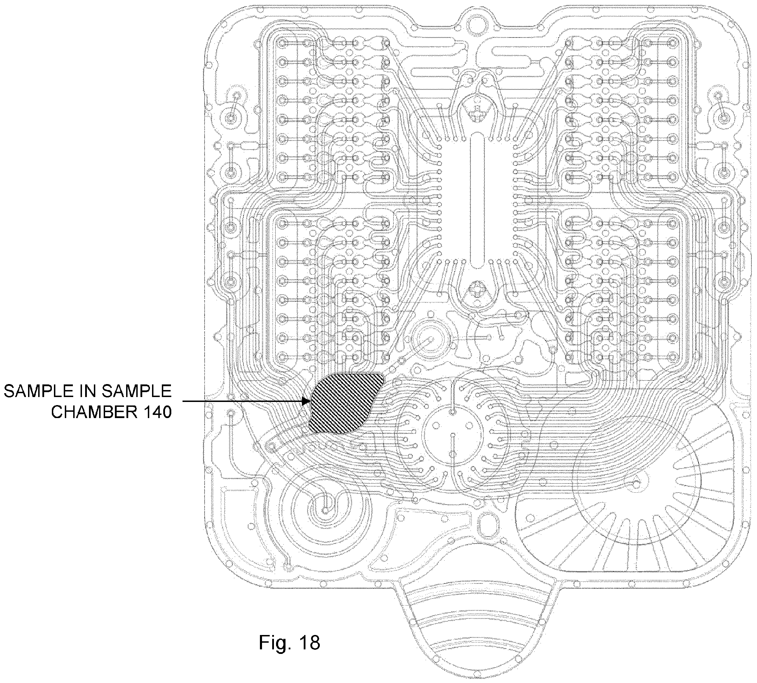

In connection with actual use, the user adds the biological sample into a sample chamber or well 140 in the substrate 100 through a sample input 230 in the top lid 200 as shown in Fig. 3A, see also Figs. 17 and 18. In an embodiment, a predefined volume of biological sample is preferably added into the sample chamber 140 through the sample input 230. In a preferred embodiment, a cap or lid 235 is attached to the sample input 230 in the top lid 200 to enclose the biological sample in the sample chamber or well 140 as shown in Fig. 3B. The cartridge 1 with the biological sample can then be inserted into an instrument (not shown) configured to load the biological sample into the microfluidic chip 500 and capture cells present in the biological sample in the microfluidic chip 500. The instrument is preferably also configured to expose the captured cells in the microfluidic chip 500 to one or multiple agents that are preferably preloaded in the substrate 100 as will be further described herein. The response, preferably phenotypic response, of the cells in the microfluidic chip 500 to the one or more agents can then be monitored and analyzed by the instrument.

The substrate 100 optionally, but preferably, comprises a grip or handle 101 that simplifies manual handling of the cartridge 1, such as when inserting the cartridge 1 into the instrument and removing the cartridge 1 from the instrument.

The instrument is preferably configured to operate by applying pressurized fluid, preferably pressurized gas and more preferably pressurized air to pressure ports 108A to 108H in the substrate 100, see Fig. 4A. The application of pressurized fluid into these pressure ports 108A to 108H is used to open the medium blister, transport culture medium throughout the substrate 100 and towards the microfluidic chip 500, transport the biological sample throughout the substrate 100 and towards the microfluidic chip 500 to therein capture any cells present in the biological sample.

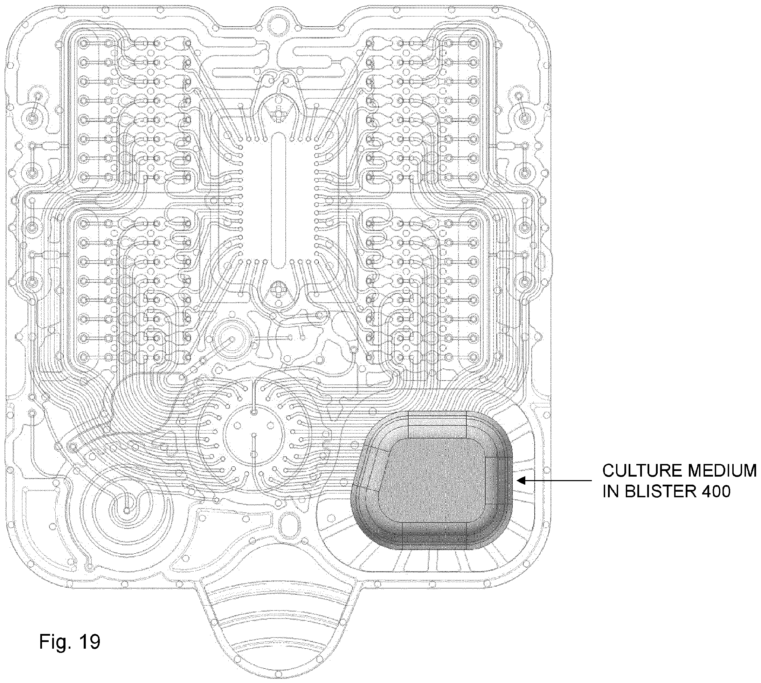

As is schematically shown in Figs. 4A, 4B, 5 and 19, the medium blister 400 is provided in the blister chamber or well 110, also referred to as blister pocket herein. In a preferred embodiment, the blister chamber 110 comprises a central recess 111 and a peripheral substantially flat chamber bottom 112. In such a case, the medium blister 400 is preferably attached to the peripheral flat chamber bottom 112 either by welding, such as laser welding, or by an adhesive, such an adhesive tape applied along the peripheral part of the bottom surface of the medium blister 400. The central recess 111 comprises a drainage hole 113 and one or more cutters 114. The central recess 111 is sufficient deep so that when the medium blister 400 is arranged in the blister chamber 110, the cutter 114 is spaced a distance from the bottom surface of the medium blister 400.

The medium blister 400 comprises culture medium to be transported to the microfluidic chip 500 and used therein for the culturing of cells captured in the microfluidic chip 500 from the biological sample. Hence, the type of culture medium contained in the medium blister 400 is preferably selected based on the type of cells to be captured.

The medium blister 400 may be manufactured from various materials including metals and/or plastics. A typical example of medium blister 400 is an aluminum blister coated with a plastic. The bottom of the medium blister 400 is preferably made of thin foil configured to by punctured and opened by the cutter 114. An illustrative, but non-limiting, example of such a foil is an aluminum foil.

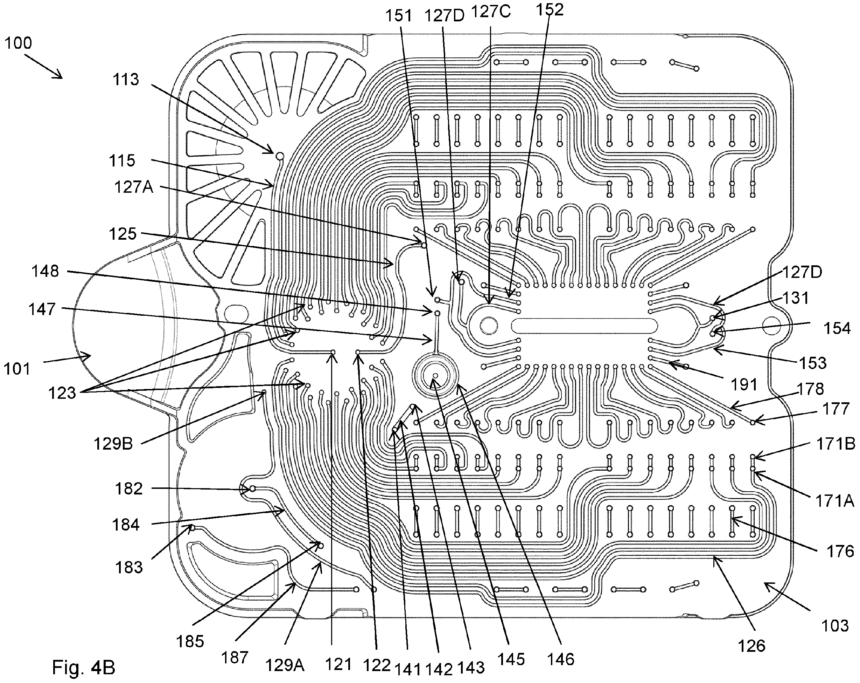

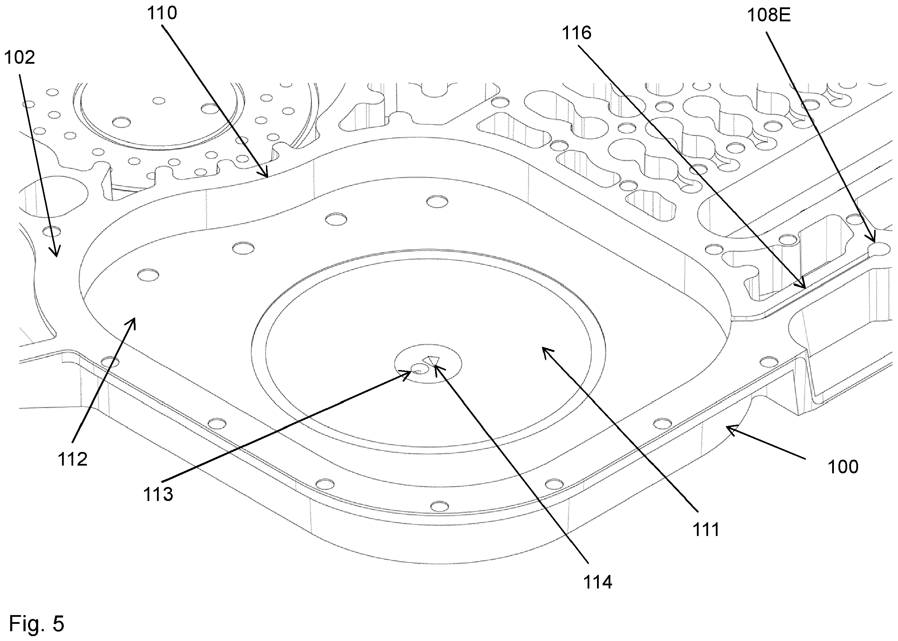

The medium blister 400 is opened by applying a fluid pressure, preferably a gas pressure, at a pressure port 108E that is fluid connection with the blister chamber 110. In more detailed, pressurized fluid, preferably pressurized gas, and more preferably pressurized air is introduced by the instrument into the pressure port 108E and is flown into the blister chamber 110 via a pressure channel 116 interconnecting the pressure port 108E and the blister chamber 110, see Fig. 5. The pressurized fluid is introduced in the blister chamber 110 between the top lid 200 and the upper surface of the medium blister 400. The pressurized fluid will thereby press the medium blister 400 downwards towards the central recess 111 causing the cutter 114 to engage and penetrate the bottom of the medium blister 400. The culture medium contained in the opened medium blister 400 is pushed by the applied pressure through the drainage hole 113 and is transported in a channel 115 in the bottom surface 103 of the substrate 100 towards an entrance hole 121 in a medium valve chamber or well 120, also referred to as medium valve pocket herein, see Figs. 4B, 6, 17 and 21.

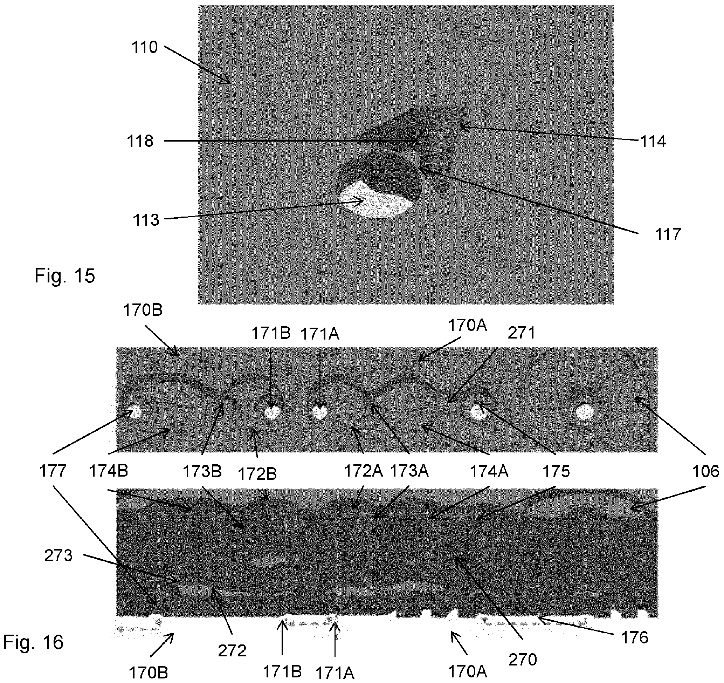

Fig. 15 is a close-up view of a portion of the blister chamber 110 showing the drainage hole 113 and an embodiment of the cutter 114. In this embodiment, the cutter 114 preferably comprises a recess or channel 118 in the side 117 of the cutter 114 facing the drainage hole 113. This recess or channel 118 guides culture medium from the opened medium blister 400 into the drainage hole 113. Hence, a more efficient emptying of the medium blister 400 and pushing of the culture medium into the drainage hole 113 is achieved if the cutter 114 comprises such a guidance or drainage recess or channel 118.

Another feature of the cutter 114 that facilitates emptying of culture medium into the drainage hole 113 is to have a curved side 117 of the cutter 114 facing the drainage hole 113. As is schematically shown in Fig. 15, the side 117 facing the drainage hole 113 is slightly curved or arced around a portion of the drainage hole 113. This shape of the side 117 will facilitate the flow of culture medium from the opened medium blister 118 along the side 117 of the cutter 114 and down into the drainage hole 113.

With reference to Figs. 4A, 4B, 6 and 21, the medium valve chamber 120 is designed to distribute the culture medium entering the entrance hole 121 to a number of medium reservoirs 170 and to a back channel reservoir 130 and additionally prevent back flow of culture medium through the substrate 100. The bottom of the medium valve chamber 120 comprises the central entrance hole 121 and a back channel hole 122 and a number of medium reservoir holes 123 circumferentially arranged around the entrance hole 121. The medium valve chamber 120 preferably comprises a respective medium reservoir hole 123 per medium reservoir 170 in the substrate 100.

The peripheral or circumferential portion 124 of the bottom surface of the medium valve chamber 120 is preferably flat to enable a culture medium valve 420 to be attached to this peripheral portion 124 of the bottom surface, such as by welding, preferably by laser welding. The culture medium valve 420 is preferably in the form of a disc made of a substantially flexible material, such a thermoplastic elastomer (TPE). When culture medium enters the entrance hole 121 the culture medium is pushed in between the bottom surface of the medium valve chamber 120 and the culture medium valve 420 that is flexed upwards by the pressure applied at the pressure port 108E. The culture medium is further pushed into the medium reservoir holes 123 and into the back channel hole 122.

The redirection of culture medium from the entrance hole 121 to the medium reservoir holes 123 and the back channel hole 122 is, in an embodiment, facilitated by application of a low pressure (about 2 bar) to the upper side of the culture medium valve 420, i.e., between the culture medium valve 420 and the top lid 200. This low pressure is applied by the instrument through the pressure port 108G and a pressure channel 129A in the bottom surface 103 of the substrate 100 and into the pressure inlet 129B in the medium valve chamber 120, see Figs. 4A and 4B.

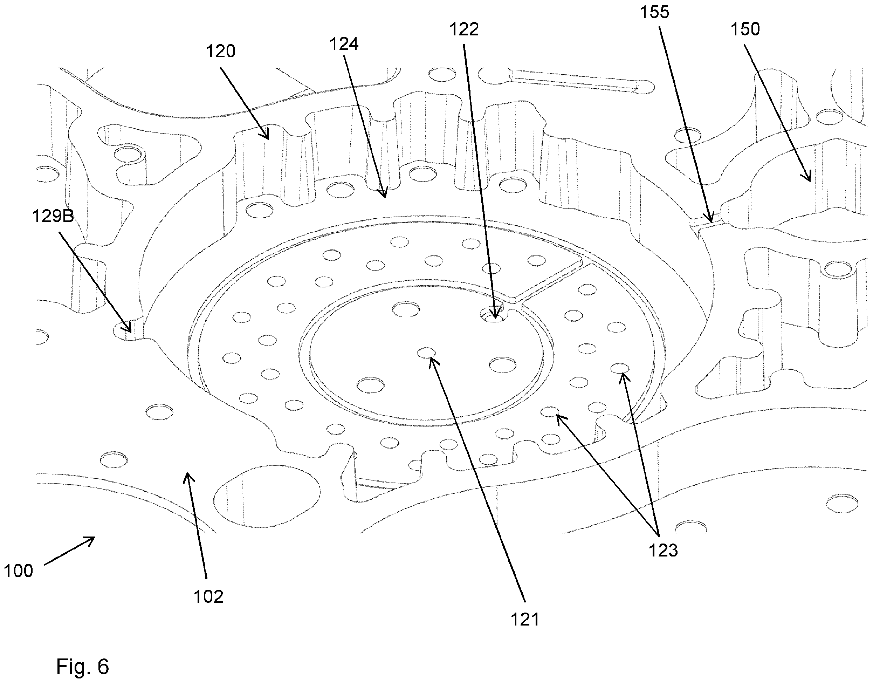

In an embodiment, the bottom surface of the medium valve chamber 120 comprises at least one recessed circular channel 129 connected to the back channel hole 122. This at least one recessed circular channel 129 guides the culture medium to the back channel hole 122 and facilitates emptying of the medium valve chamber 120 of culture medium, see Fig. 6.

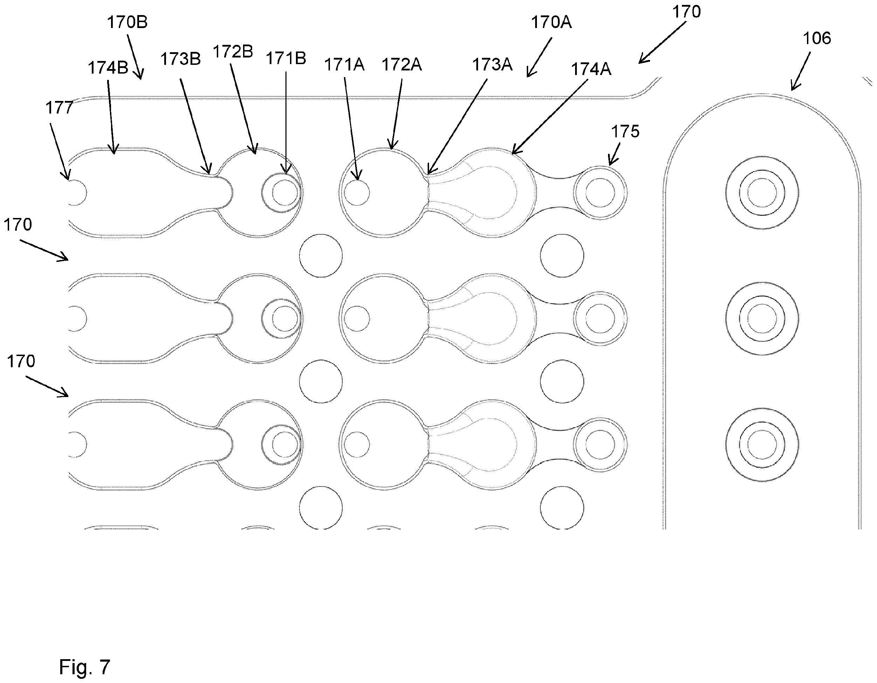

The culture medium is, in an embodiment, initially transported from the medium valve chamber 120 to the medium reservoirs 170 to fill each medium reservoir 170 with an equal and defined volume of culture medium prior to transporting culture medium to the back channel reservoir 130, see Figs. 7, 17 and 21. This sequential filling of the back channel reservoir 130 after the filling the medium reservoirs 170 can be controlled by applying a fluid pressure, preferably a gas pressure, and more preferably an air pressure to a pressure port 108A in fluid connection with the back channel reservoir 130. Initially, the pressure applied at the pressure port 108A implies that the culture medium will be exposed to a higher flow resistance in the channels interconnecting the back channel hole 122 with the back channel reservoir 130 as compared to any flow resistance in the channels interconnecting the respective medium reservoir holes 123 with the medium reservoirs 170.

The medium valve chamber 120 with the culture medium valve 420 provides several functions during the filling process of the substrate 100. Firstly, the medium valve chamber 120 and the culture medium valve 420 operates as a 1-to-(*N*+1) valve, where *N* represents the number of medium reservoir holes 123 and the number of medium reservoirs 170 in the substrate 100. In other words, the medium valve chamber 120 redirects the inflow of culture medium through the entrance hole 121 into the *N* medium reservoir holes 123 and the back channel hole 122.

The culture medium valve 420 additionally operates as a check valve preventing flow of culture medium from the medium reservoirs 170 and/or the back channel reservoir 130 back into the medium valve chamber 120 by the application of a fluid pressure, preferably gas pressure, and more preferably an air pressure on the top of the culture medium valve 420. This pressure forces the culture medium valve 420 towards the bottom surface of the medium valve chamber 120 and thereby closes the holes 121, 122, 123 in the bottom of the medium valve chamber 120. As a consequence, the holes 121, 122, 123 become sealed and any undesired cross-talk between the medium reservoirs 170 and the back channel reservoir 130 is thereby prevented. This is important to prevent culture medium entering one medium reservoir 170 that may comprise one agent from mixing with culture medium entering another medium reservoir that may comprise another agent or the same agent but at a different amount.

The closure of the medium valve chamber 120 with the culture medium valve 420 is achieved by applying a pressure at the pressure port 108G causing pressurized fluid, preferably pressurized gas, and more preferably pressurized air to be pushed through the pressure channel 129A and enter the medium valve chamber 120 through the pressure inlet 129B, see Figs. 4A, 4B and 6.

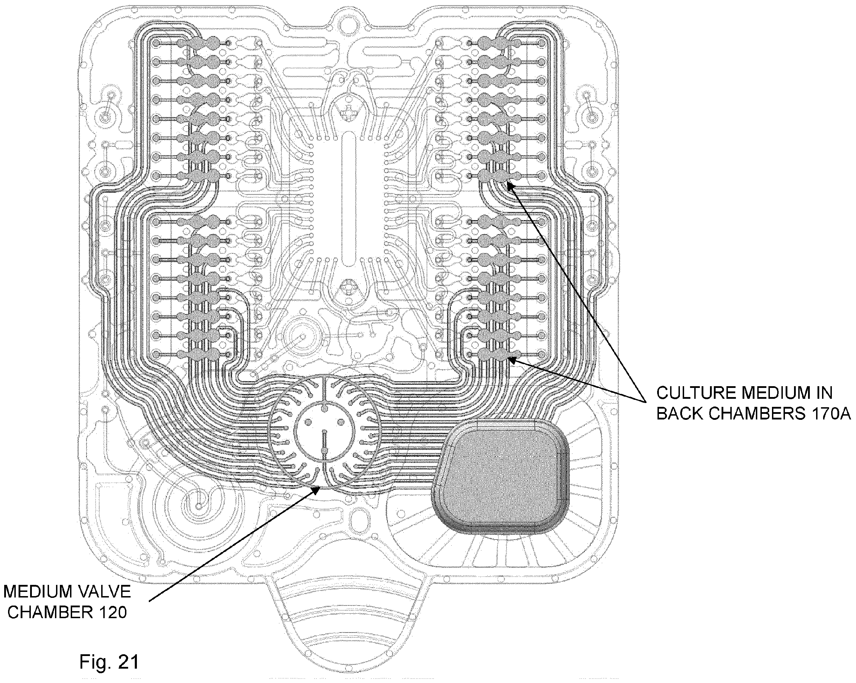

With reference to Figs. 4A, 4B and 7, each medium reservoir hole 123 is connected to respective entrance holes 171A, 171B of a medium reservoir 170 through a respective medium channel 126 arranged in the bottom surface 103 of the substrate 100. The culture medium is pushed through the medium channels 126 and into the medium reservoirs 170 through these entrance holes 171A, see also Fig. 21.

The medium reservoirs 170 are designed to house a predefined volume of culture medium and, in particular, that each medium reservoir 170 of the plurality of medium reservoirs 170 will contain the same or at least substantially the same predefined volume of culture medium. Accordingly, the medium reservoirs 170 are designed to facilitate removal of air inside the medium reservoirs 170 during filling to thereby prevent or at least significantly reduce the risk of trapping any air bubbles in the medium reservoirs 170 that, if present, would interfere with filling the medium reservoirs 170 with the predefined volume of culture medium.

The medium reservoirs 170 are further designed to be preloaded with agents to be mixed with the culture medium to thereby dissolve or disperse the agents in the culture medium. By having a plurality of different medium reservoirs 170 it is possible to include different agents in different medium reservoirs 170 and/or include the same agent in different medium reservoirs 170 but in different amounts to thereby achieve different concentrations of the agent when dissolved or dispersed in the predefined volume of culture medium. For instance, in a substrate 100 having 32 different medium reservoirs 170 as schematically shown in Fig. 4A, five different agents at five different concentrations could be preloaded into 25 of the 32 medium reservoirs 170 with the remaining seven medium reservoirs 170 used as various controls, such as not preloaded with any agents or preloaded with one or more control chemicals or agents.

In an embodiment, each medium reservoir 170 comprises two separate but interconnected chambers or wells, denoted back chamber or well 170A and front chamber or well 170B herein. As is more clearly seen in Fig. 4B, each medium channel 126 from the medium valve chamber 120 is preferably connected to two entrance holes 171A, 171B one for the back chamber 170A and one for the front chamber 170B. The front chambers 170B are in fluid connection with the microfluidic chip 500 and thereby, due to the minute dimensions of the microfluidic channels in the microfluidic chip 500, exert a higher flow resistance to the culture medium in the medium channels 126 than the flow resistance exerted by the back chambers 170A. This difference in flow resistance will force the culture medium flowing in the medium channels 126 to enter the entrance holes 171A of the back chambers 170A but not into the entrance holes 171B of the front chambers 170B.

The back chamber 170A of the medium reservoirs 170 preferably comprises a pillar chamber or well 172A with the entrance hole 171A. The back chamber 170A also comprises a back chamber or well part 174A with a waist 173A or narrow passage between the pillar chamber 172A and the back chamber part 174A. The waist 173A restricts the culture medium flow in the back chamber 170A so that when the culture medium enters through the entrance hole 171A the culture medium will first fill up the pillar chamber 172A prior to flowing past the waist 173A and into the back chamber part 174A. The initial filling part of the back chamber 170A thereby creates a pillar of culture medium in the pillar chamber 172A prior to continuing filling the back chamber part 174A. This sequential filling of the back chamber 170A in two steps facilitates venting air present in the back chamber 170A from the pillar chamber 172A into the back chamber part 174A and further into a passage 175 that interconnects, together with a channel 176 arranged at the bottom surface 103 of the substrate 100, the back chambers 170A with the membrane chambers 106. Accordingly, the risk of unintentional entrapment of small air bubbles in the back chamber 170A during the filling process is minimized, thereby enabling a correct filling of the back chamber 170A with a predefined volume of culture medium.

Once the pillar chambers 172A have been filled with culture medium, the culture medium is pushed through the waists 173A and into the back chamber parts 174A. When the pillar chambers 172A and the back chamber parts 174A are both filled with culture medium, any excess the culture medium will be pushed into channels 271, see Fig. 16, interconnecting the back chamber parts 174A and the passages 175 above partitions or walls 270 between the back chamber parts 174A and the passages 175. The excess culture medium is then further pushed through the passages 175 and channels 176 towards the membrane chambers 106.

The membrane chambers 106 comprise each a gas permeable membrane 600 configured to enable a gas, such as air, to pass through the gas permeable membrane 600 but restrict culture medium from passing through the gas permeable membrane 600. Hence, air present in the back chambers 170A is, during filling with culture medium, pushed through the gas permeable membranes 600 and further through pressure ports 108B, 108D in fluid connection with the membrane chambers 106. This means that any air present in the back chambers 170A is vented out therefrom during the filling process. When the back chambers 170A are filled with culture medium any excess culture medium is pressed through the passages 175 and the channels 176 into the membrane chambers 106. However, when culture medium contacts the gas permeable membranes 600 present in the membrane chambers 106 it is prevented from flowing any further and the filling process of the back chambers 170A is completed. Hence, the gas permeable membranes 106 exert a counter force onto the culture medium that is higher than the force caused by applying a pressure onto the culture medium valve 420 in the medium valve chamber 120. The gas permeable membrane 600 also prevents culture medium in one back chamber 170A from entering another back chamber 170A through the membrane chamber 106 and the passages 175 and the channels 176.

When the back chambers 170A in the medium reservoirs 170 have been filled with the predefined volume of culture medium, the counter pressure applied to the back channel reservoir 130 via the pressure port 108A is released, see Fig. 17. This causes remaining culture medium to be pushed from the medium valve chamber 120 through the back channel hole 122 and a medium channel 125 in the bottom surface 103 of the substrate 100, see Fig. 4B. In addition, when the medium reservoirs 170 are filled, there may still be a volume of culture medium in the medium blister 400. This volume of culture medium is then emptied, though the medium valve chamber 120 into the back channel reservoir 130.

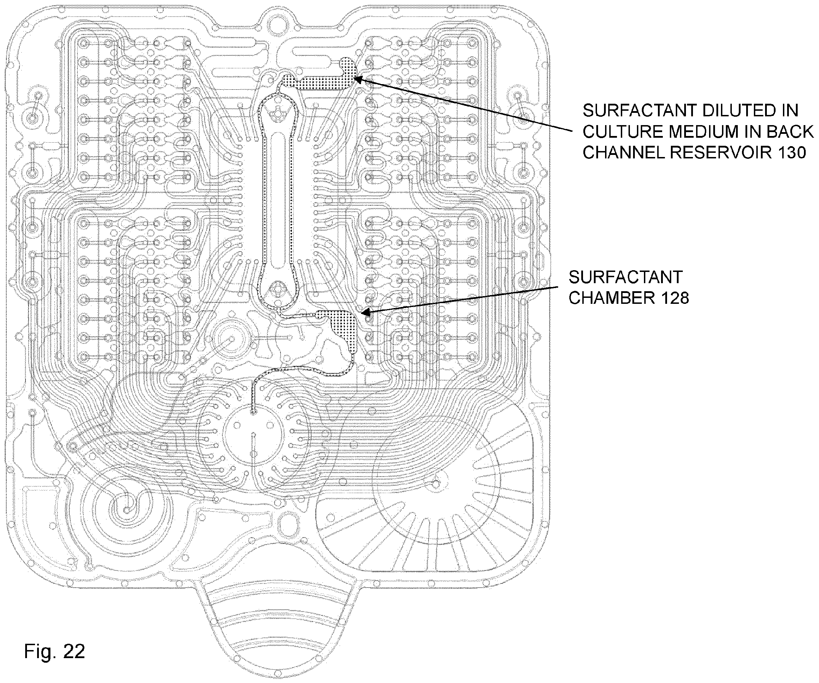

In a preferred embodiment and with reference to Figs. 4A, 4B, 8, 17 and 22, the culture medium passes through a surfactant chamber or well 128 prior to reaching the back channel reservoir 130. This surfactant chamber 128 is preferably preloaded with a surfactant that is mixed with and dissolved in the culture medium in the surfactant chamber 128. The surfactant is preferably dissolved or at least dispersed in the culture medium to be used for wetting microfluidic channels in the microfluidic chip 500 prior to transporting the biological sample into the microfluidic chip 500.

Any surfactant that can be dissolved or at least dispersed in a culture medium and that enables a wetting of the microfluidic channels in the microfluidic chip 500 can be used according to the invention and preloaded in the surfactant well 128. Non-limiting, but illustrative, examples of surfactants are nonionic surfactants, such poloxamers. Poloxamers are triblock copolymers composed of a central hydrophobic chain of polyoxypropylene (poly(propylene oxide)) flanked by two hydrophilic chains of polyoxyethylene (poly(ethylene oxide)). Examples of poloxamers that can be used as surfactants include poloxamers sold under the trade name PLURONIC^{®}, such as PLURONIC^{®} F108.

The surfactant chamber 128 is preferably designed to have an entrance hole 127A and an exit hole 127B arranged in the upper part of the surfactant chamber 128 to thereby force culture medium entering the surfactant chamber 128 from the medium channel 125 and through the entrance hole 127A to contact with the surfactant preloaded at the bottom of the surfactant chamber 128 prior to exiting the surfactant chamber 128 through the exit hole 127B. This enables the surfactant to be dissolved or dispersed in the culture medium prior to exiting the surfactant chamber 128. The culture medium with surfactant is then transported through a channel system 127C, 127D in the bottom surface 103 of the cartridge 103, and a first channel 551 in the carrier chip 550 before reaching an entrance hole 131 to the back channel reservoir 130.

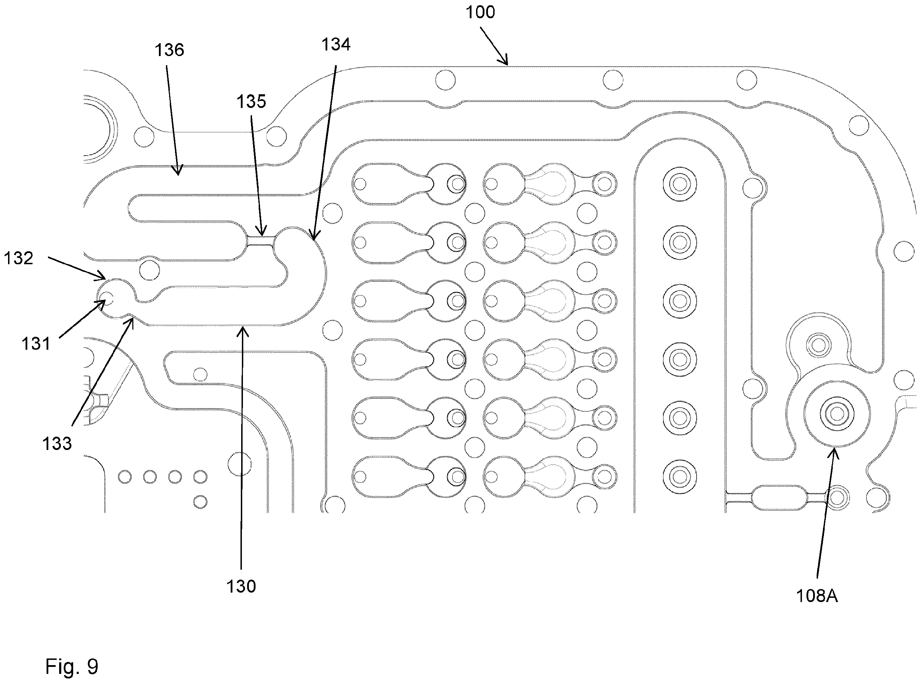

The back channel reservoir 130, is in an embodiment and with reference to Figs. 4A, 4B and 9, designed in a similar way to the back chamber 170A of the medium reservoir 170 with the entrance hole 131 entering a pillar chamber or well 132. The pillar chamber 132 is connected to a back chamber or well 134 through a narrow waist 133. The back chamber 134 is also connected to a back channel waste 136 through a passage 135.

Culture medium with surfactant enters the back channel reservoir 130 through the entrance hole 131 and initially fills the pillar chamber 132 while venting air through the waist 133 into the back chamber 134 and the passage 135 and into the back channel waste 136. The back channel waste 136 is in fluid connection with a pressure port 108A thereby allowing air entering the back channel waste 136 to leave through the pressure port 108A. Once the culture medium with surfactant forms a pillar and completely fills the pillar chamber 132 the culture medium with surfactant is pushed through the waist 133 and into the back chamber 134. Any excess culture medium with surfactant can, when also the back chamber 134 is filled, enter the back channel waste 136 through the passage 135. Hence, the back channel waste 136 is designed to have volume that can house any surplus culture medium with surfactant that is pushed into back channel reservoir 130.

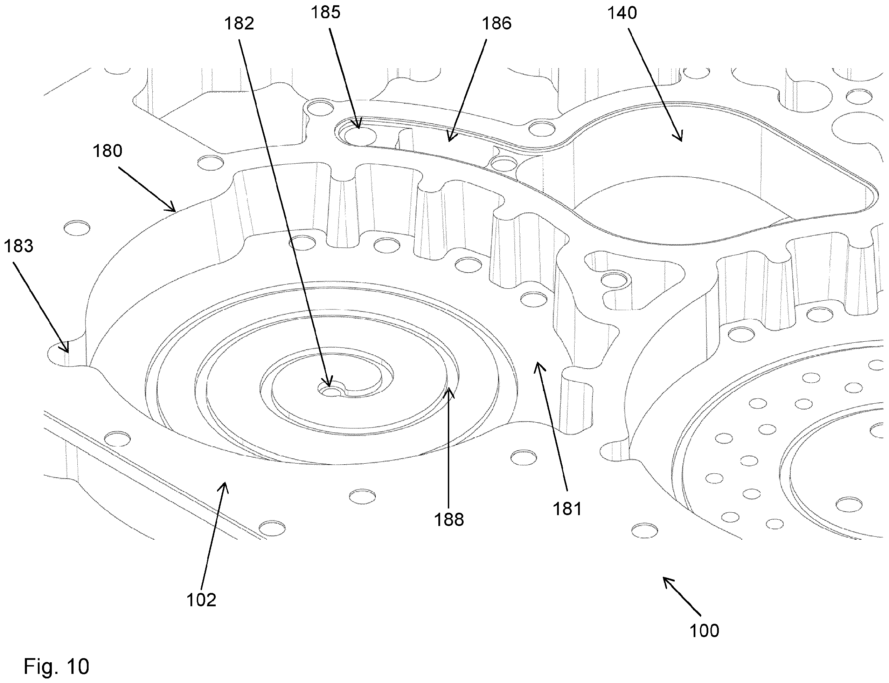

The substrate 100 preferably comprises a dome pump chamber or well 180 configured to include the dome shaped pump 410, see Figs. 4A, 4B and 10. The dome shaped pump 410 is attached to the bottom surface of the dome pump chamber 180, such as by welding, preferably laser welding, the circumferential part of the dome shaped pump 410 to the peripheral part 181 of the bottom surface of the dome pump chamber 180. The dome shaped pump 410, when attached to the bottom surface of the dome pump chamber 180, encloses a defined volume of air.

When a pressure is applied to the medium blister 400 in the blister chamber 110 through the pressure port 108E, a pressure is, preferably simultaneously, applied to the top of the dome shaped pump 410 through the pressure port 108H via a pressure channel 187 and a pressure inlet 183 in the dome pump chamber 180, see Fig. 17. The applied pressure presses the dome shaped pump 410 downwards towards the bottom surface of the dome pump chamber 180. The predefined volume of air contained by the dome shaped pump 410 in the dome pump chamber 180 is then pressed through a drainage hole 182 preferably placed in the center of the dome pump chamber 180. The bottom surface optionally comprises a coiled recess 188 to guide the pressed air into the drainage hole 182.

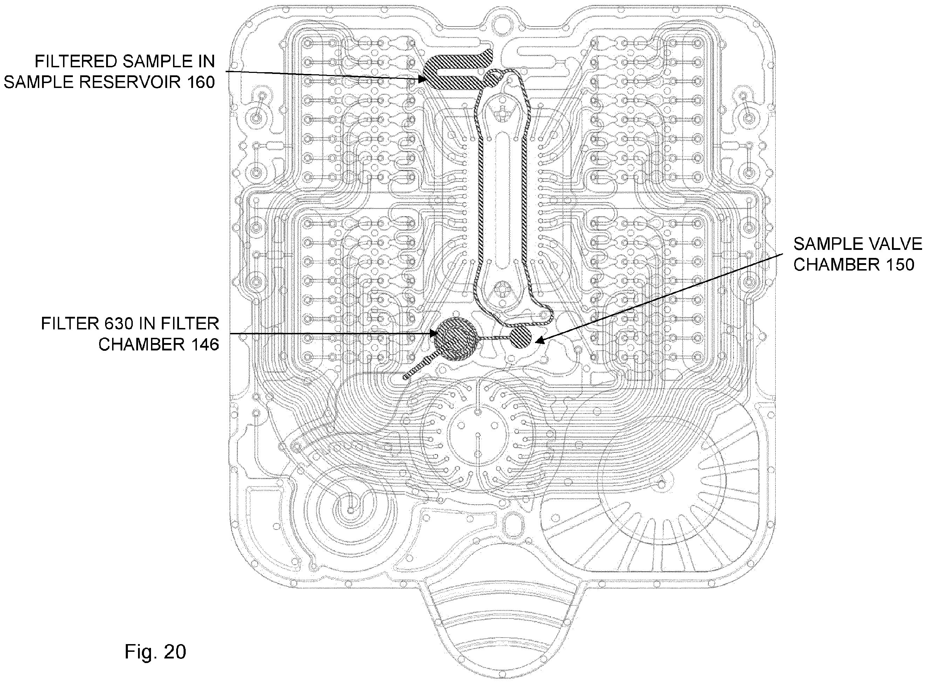

The drainage hole 182 is in fluid connection with an entrance hole 185 in the sample chamber or well 140 via an air channel 184 in the bottom surface 103 of the substrate 100. The predefined volume of air entering the sample chamber 140 though the entrance hole 185 and an air passage 186 in the top surface 102 of the substrate 100 pushes a controlled volume of biological sample from the sample reservoir 140 through a drainage hole 141 and sample transport system towards a filter chamber or well 146, also denoted filter pocket herein. In an embodiment, the transport system comprises a channel 142 in the bottom surface 103 of the substrate 100, a channel 143 from the bottom surface 103 to the top surface 102 of the substrate 100, and a channel 144 in the top surface 102 of the substrate 100. The biological sample is thereby pushed into an entrance hole 145 into the filter chamber 146 and through the sample filter 630 arranged in the filter chamber 146, see also Figs. 17 and 20. The sample filter 630 is arranged in the filter chamber 146 to filter away any larger debris, dust or dirt present in the biological sample but allow any cells present in the biological sample to pass through the sample filter 630. The filtered biological sample is further transported through a channel 147 in the bottom surface 103 of the substrate 100 into an entrance hole 148 of a sample valve chamber or well 150, also denoted sample valve pocket herein, see also Fig. 20.

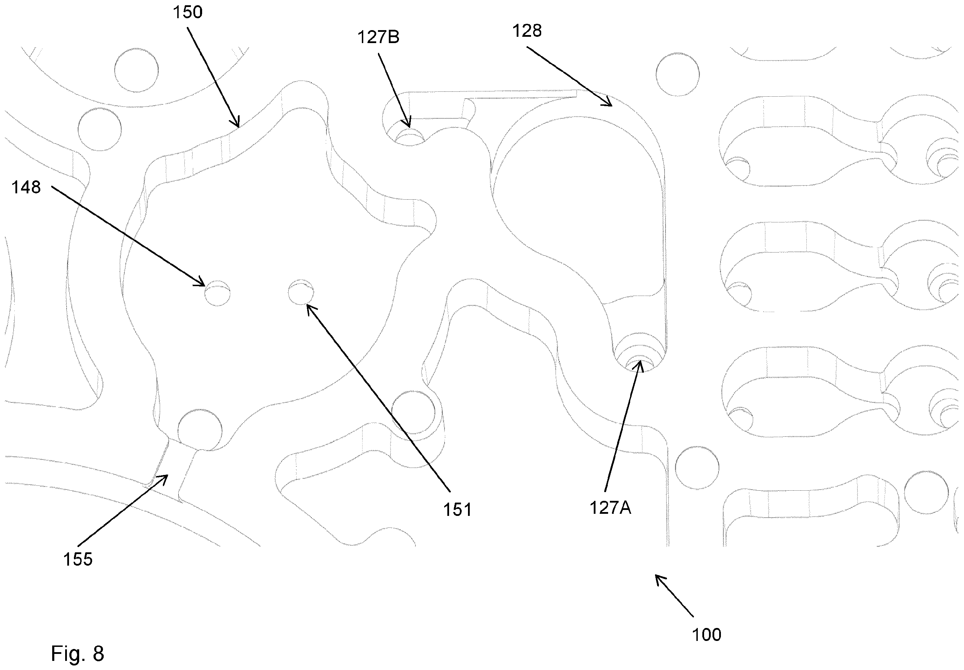

With references to Figs. 4A, 4B and 8, the sample valve chamber 150 comprises a sample valve 430 that is attached to the sample valve chamber 150, preferably by welding, such as laser welding, the circumferential portion of the sample valve 430 to the peripheral portion of the bottom surface of the sample valve chamber 150. The sample valve 430 is made of a flexible material, such as in the form of a TPE disc, and acts as check valve. The sample valve chamber 150 comprises, in addition to the entrance hole 148, a drainage hole 151 in the bottom surface of the sample valve chamber 150. Filtered biological sample entering the valve chamber through the entrance hole 148 is further pushed through this drainage hole 151 and is transported by a transport system to a sample reservoir 160. The transport system comprises, in an embodiment, a first T-shaped channel 152 arranged in the bottom surface 103 of the substrate 100, a second channel 553 in the chip carrier 550 and a second T-shaped channel 153 arranged in the bottom surface 103 of the substrate 100 and ending at an entrance hole 154 to the sample reservoir 160, see Figs. 17 and 20.

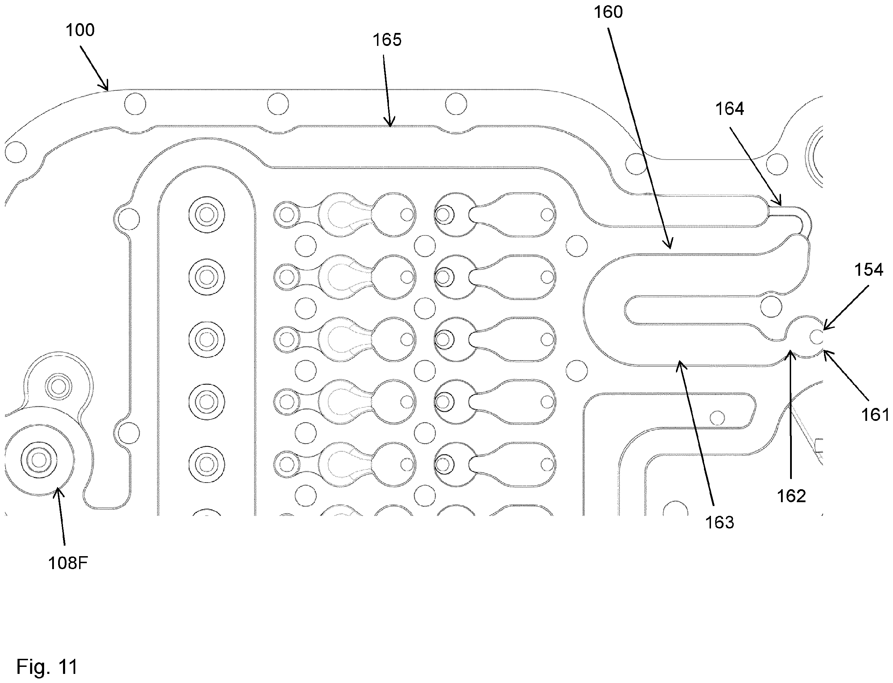

In an embodiment and with reference to Figs. 4A, 4B and 11, the sample reservoir 160 is designed similar to the back channel reservoir 130 with the entrance hole 154 in a pillar chamber or well 161 that is initially filled with the filtered biological sample while venting air through a waist 162 into a back chamber 163 and a passage 164 and into a sample waste 165. The sample waste 165 is in fluid connection with a pressure port 108F thereby allowing air entering the sample waste 165 to leave through the pressure port 108F. Once the filtered biological forms a pillar and completely fills the pillar chamber 161 the filtered biological sample is pushed through the waist 162 and into the back chamber 163. Any excess filtered biological sample can, when also the back chamber 163 is filled, enter the sample waste 165 through the passage 164. Hence, the sample waste 165 is designed to have volume that can house any surplus filtered biological sample that is pushed into sample reservoir 160.

As previously discussed herein, the sample valve chamber 150 is in fluid communication with the medium valve chamber 120 via a passage 155 between the chambers 120, 150. Accordingly, when a pressure is applied to the medium valve chamber 120 to press the culture medium valve 420 therein towards the bottom surface of the medium valve chamber 120 and prevent back flow of culture medium from the back channel reservoir 130 and the medium reservoirs 170 towards the medium valve chamber 120 the pressure is also applied to the sample valve chamber 150 to press the sample valve 430 therein towards the bottom surface of the sample valve chamber 150. The sample valve 430 thereby prevents back flow of the filtered biological sample from the sample reservoir 160 towards the sample valve chamber 150.

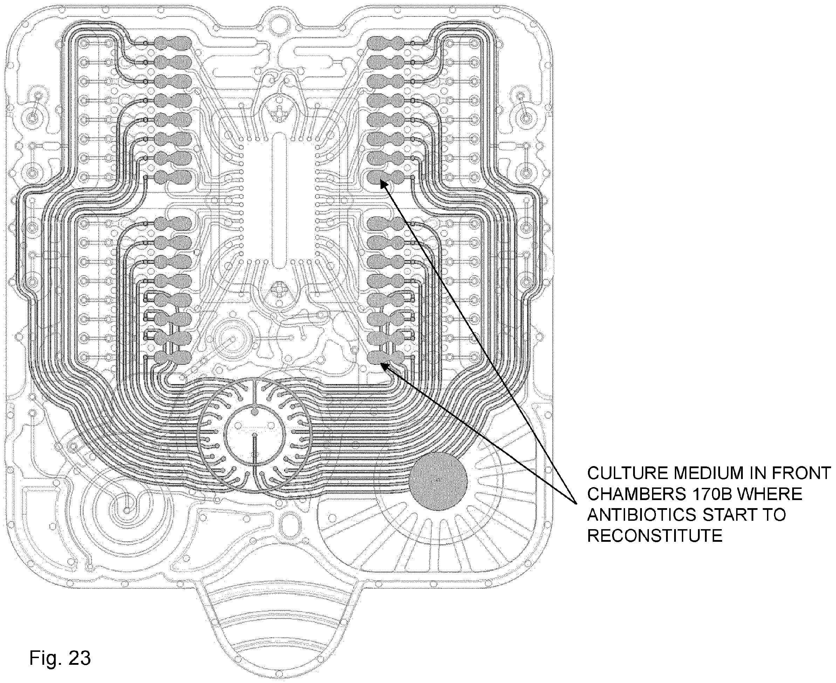

With reference to Figs. 4A, 4B and 7, the predefined volume of culture medium that is contained in the back chambers 170A and the passages 175 and channels 176 is then pressed through the entrance holes 171A of the back chambers 170A and into the entrance holes 171B of the front chambers 170B by application of an overpressure onto the membrane chambers 106 through the pressure ports 108B, 108D, see Figs. 17 and 23.

In an embodiment, the front chambers 170B are designed in a similar way to the back chamber 170A with a pillar chamber or well 172B, a waist 173B and a front chamber part 174B. The culture medium enters the front chambers 170B through the entrance holes 171B and starts to fill the pillar chamber 172B while venting air through the waists 173B, front chamber parts 174B and a drainage hole 177 connected to the microfluidic chip 500 through a respective channel 178 in the bottom surface 103 of the substrate 100. The culture medium initially fills the pillar chambers 172B forming a pillar before flowing into the front chamber parts 174B through the waists 173B.

The internal volume of the front chambers 170B is preferably at most equal to, and more preferably smaller, than the internal volume of the back chambers 170A. This means that the front chambers 170B can be fully filled with the culture medium previously filled in the back chambers 170A.

As previously disclosed herein, at least a portion of the plurality of medium reservoirs 170 comprises one or more agents. In an embodiment, an agent is preloaded in the front chamber 170B of a medium reservoir 170 and preferably in the front chamber part 174B of the front chamber 170B. In another embodiment, an agent is preloaded in the back chamber 170A of a medium reservoir 170 and preferably in the back chamber part 174A of the back chamber 170A. It is also possible to preload a portion of the agent in the front chamber 170B, such as in the front chamber part 174B, and a remaining portion of the agent in the back chamber 170A, such as in the back chamber part 174A. It is generally preferred to preload an agent in the front chamber 170B of the medium reservoir 170. However, some agents require a comparatively longer period of time in order to dissolve or at least disperse in the culture medium. In such a case, it may be advantageous to preload all or at least a portion of the agent in the back chamber 170A to thereby prolong the time at which the agent is in contact with the culture medium. Hence, in an embodiment, the agent is preloaded in the front chamber 170B, such in the front chamber part 174B, with optional complementary or additional agent preloaded in the back chamber 170A, such as in the back chamber part 174A.

In an embodiment, the front chamber 170B comprising a mixing device 273 configured to promote mixing of any preloaded agent in the front chamber 170B and the culture medium to obtain a culture medium having a substantially homogenous concentration of the agent, see Fig. 16. In an embodiment, this mixing device 273 or function is achieved by having a step 273 arranged between the drainage hole 177 and the front chamber part 174A. This means that the bottom 272 of the front chamber part 174A is lowered relative to this step 273 and relative to the pillar chamber 172B. There is, thus, an indentation in the front chamber part 174A as shown in Fig. 16, into which the agent can be loaded. When the culture medium is pushed into the front chamber 170B, the step 273 causes a turbulence inside the front chamber part 174B, which promotes efficient mixing of the agent pre-loaded at the bottom 272 and the culture medium.

At this point, the filling operation of the cartridge 1 is completed with the filtered biological sample present in the sample reservoir 160, the culture medium with surfactant in the back channel reservoir 130 and the medium reservoirs 170 comprising culture medium in the front chambers 170B and where the culture medium in at least a portion of these front chambers 170B comprises dissolved or dispersed agents.

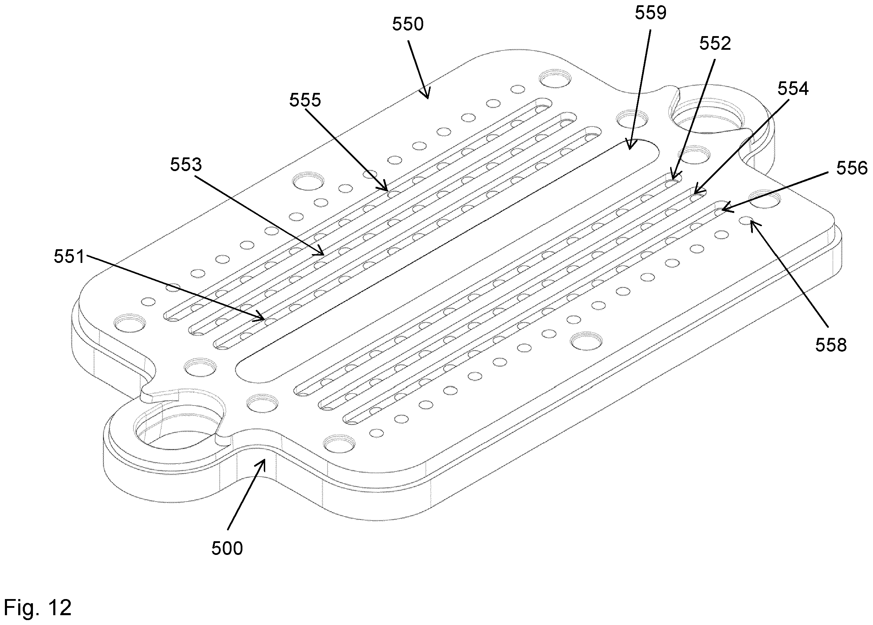

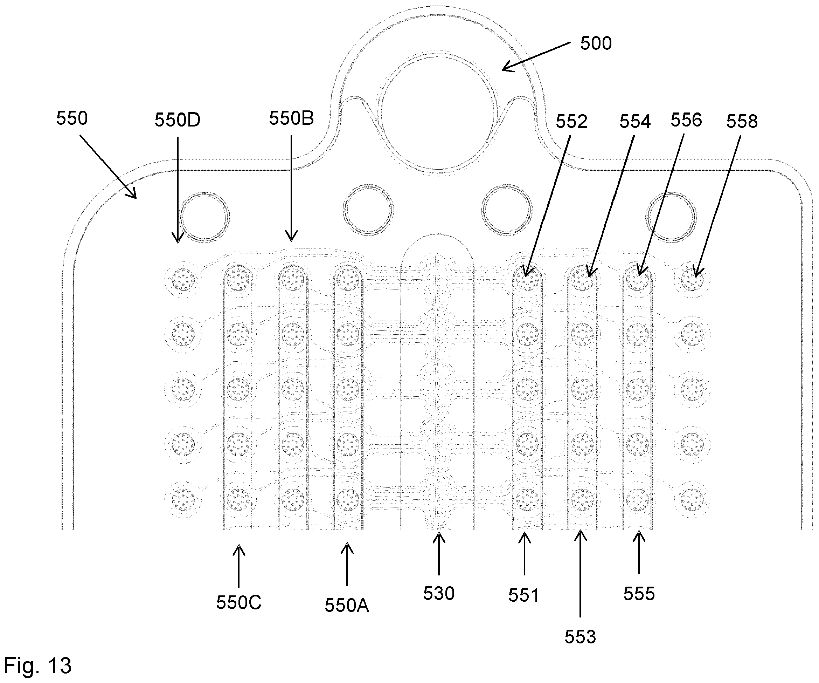

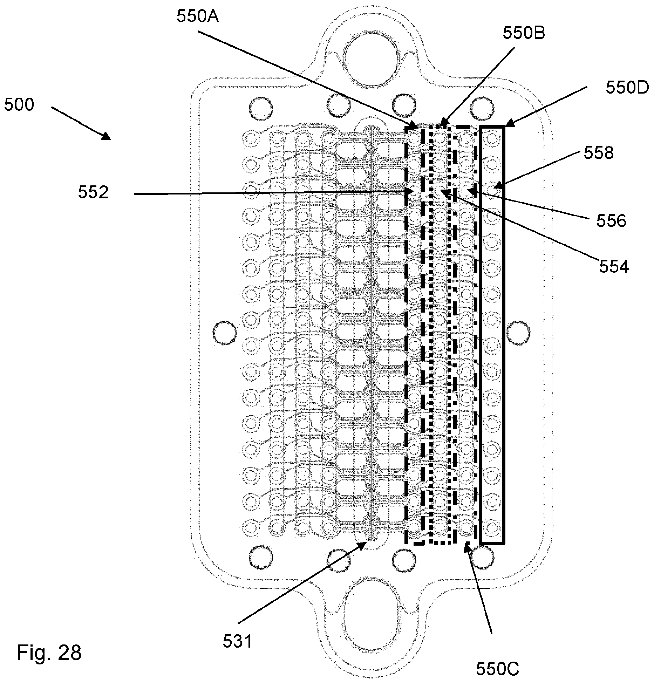

Figs. 12 to 14 and 28 illustrate the microfluidic chip 500 and the chip carrier 550 in detail. The chip carrier 550 comprises a matrix or array of entrance holes 552, 554, 556, 558 providing access to the microfluidic chip 500. This matrix preferably comprises four columns of holes and N rows of holes. The holes 552 in the first column 550A closest to a central window 559 in the chip carrier 550 are preferably interconnected via a first channel 551 running along the bottom surface of the chip carrier 550. This first channel 551 is in turn in fluid connection with the back channel reservoir 130 via a T-shaped channel 127D and the medium valve chamber 120 via the transport system 127. The holes 554 in the second column 550B are preferably also interconnected via a second channel 553 in the bottom surface of the chip carrier 550. This second channel 553 is in fluid connection with the sample reservoir 160 via the T-shaped channel 153 and to the sample valve chamber 150 via the T-shaped channel 152. The holes 556 in the third column 550C are preferably interconnected via a channel 555 in the bottom surface of the chip carrier 550. This channel is in turn in fluid connection with a waste chamber 190 via a channel 191 in the bottom surface 103 of the substrate 100. The holes 558 in the outermost, fourth column 550D are not interconnected to each other. In clear contrast, each hole 558 in this fourth column is connected via a channel 178 to a respective medium reservoir 170.

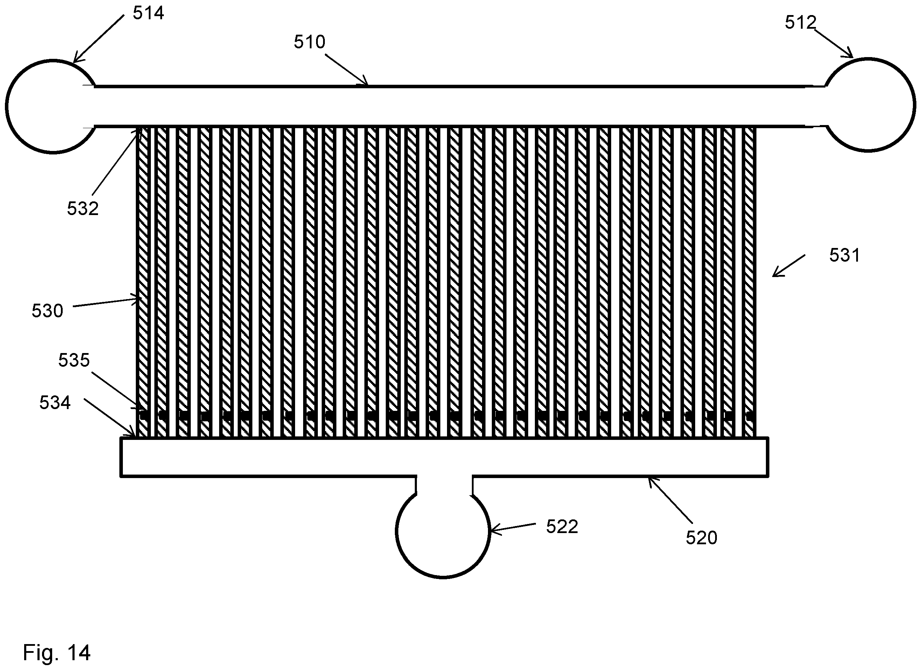

Each row of four holes 552, 554, 556, 558 of the N rows is in fluid connection with ports 512, 514, 522 of a set 531 of cell channels 530, see Figs. 14, 29 to 31. Hence, the microfluidic chip 500 comprises a plurality of such sets 531 of cell channels 530 configured to capture cells present in the biological sample. Each set 531 of cell channels 530 comprises an input channel 510 having respective first and second input ports 512, 514 in either end of the input channel 510 and an output channel 520 having an output port 522. The cell channels 530 are then arranged, preferably in parallel, between the input channel 510 and the output channel 520. Hence, each cell channel 530 in the set has a first end 532 in fluid connection with the input channel 510 and a second end 534 in fluid connection with the output channel 520. At least a portion of the cell channels 530 additionally comprises a cell block 535 configured to block cells entering the cell channel 530 from the input channel 510 from leaving the cell channel 530 and enter the output channel 520. Hence, this cell block 535 traps and captures any cells entering the cell channel 530. More information of the design of the set 531 of cell channels 530 and input and output channels 510, 520 can be found in US Patent No. 10,041,104.

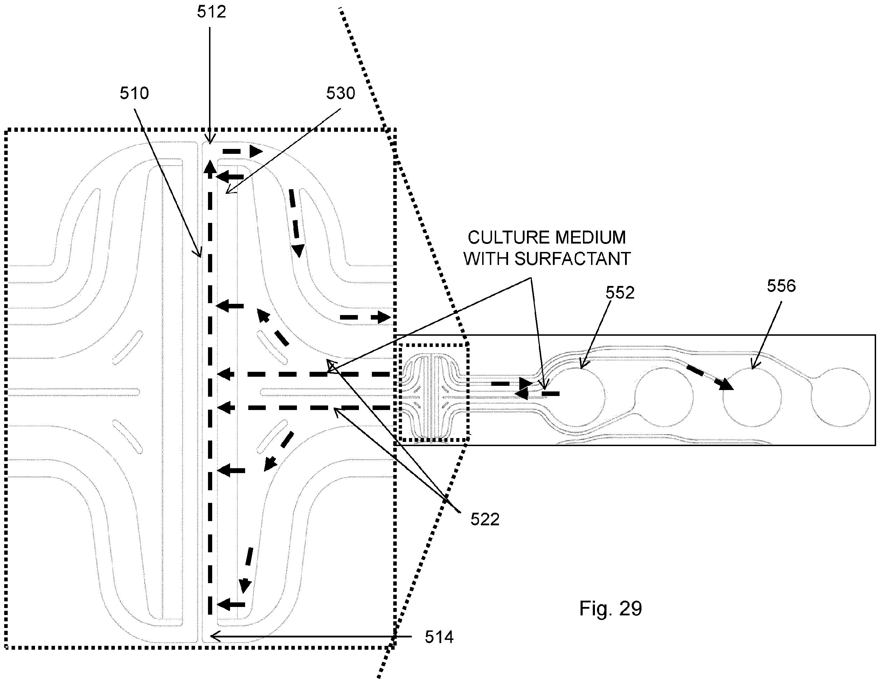

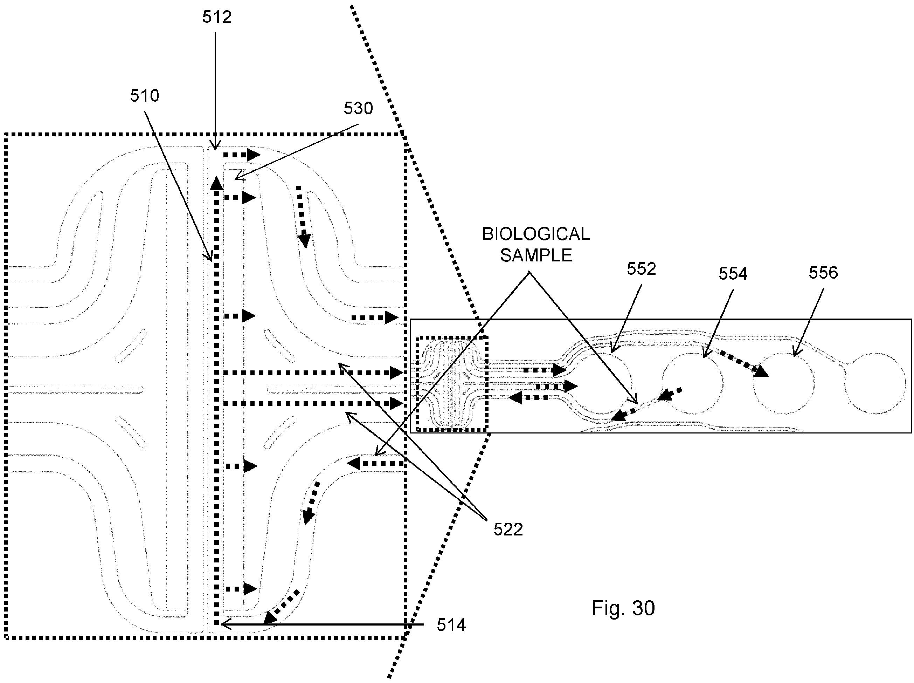

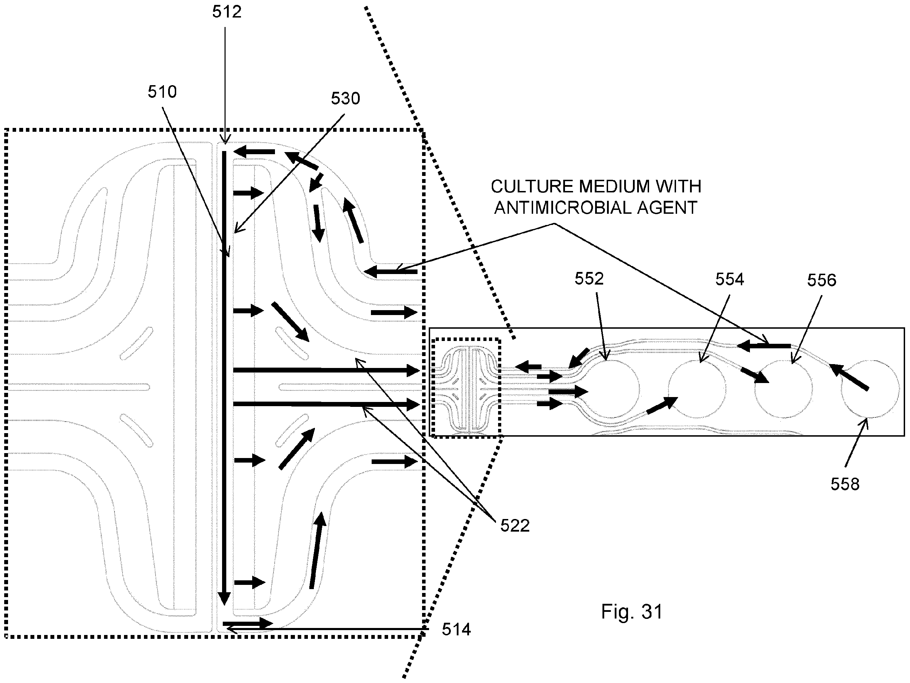

The holes 552 in the first column 550A are in fluid connection with the output ports 522 of the output channels 520, see Fig. 29, the holes 554 in second column 550B are in fluid connection with the second input ports 514 at one end of the input channels 510, see Fig. 30, whereas the holes 556, 558 in the third and fourth columns 550C, 550D are in fluid connection with the first input ports 512 at the other end of the input channels 510, see Fig. 31.

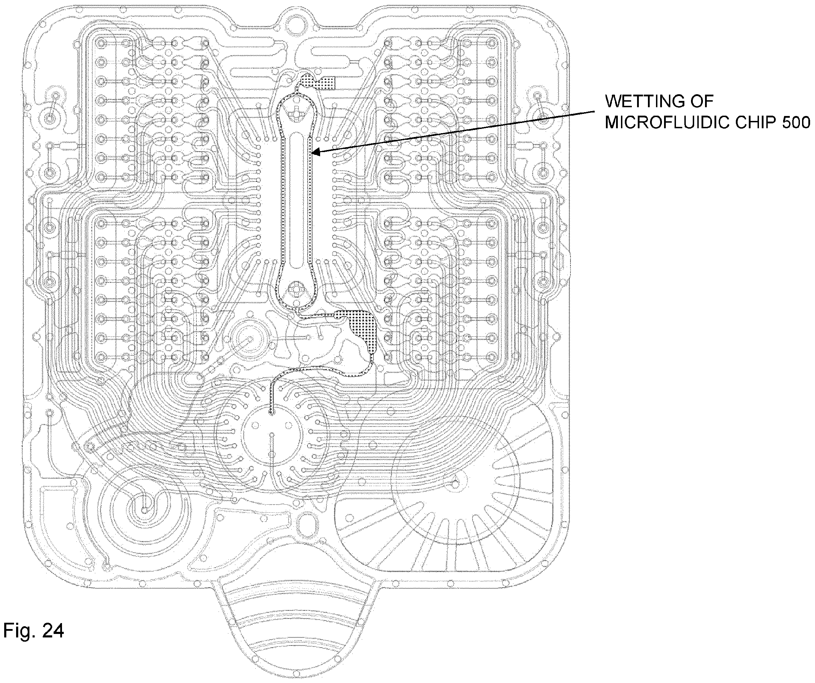

Prior to loading the filtered biological sample into the microfluidic chip 500, the microfluidic channels of the microfluidic chip 500 are preferably wetted to remove any air captured in these microfluidic channels, see Figs. 17 and 24. The wetting of the microfluidic chip 500 is performed with the culture medium comprising surfactant contained in the back channel reservoir 130. Hence, a pressure is applied at the pressure port 108A, which is in fluid connection with the back channel reservoir 130 through the back channel waste 136 and the narrow channel 135. The culture medium comprising surfactant is thereby pushed from the back channel reservoir 130 into the holes 552 and the first channel 551 in the first column 550A in the chip carrier 550, see Fig. 29. The culture medium with surfactant thereby enters the set 531 of cell channels 520 in the microfluidic chip 500 through the output ports 522 and output channels 520, flows through the cell channels 530 into the input channels 510 and out through the first input ports 512. Excess culture medium with surfactant then flows into the holes 556 in the third column 550C and further into the waste chambers 190 via the channels 191. This initial wetting of the microfluidic chip 500 removes any air trapped in the microfluidic chip 500, i.e., in the set 531 of cell channels 530 and the input and output channels 510, 520. Additionally, the surfactant dissolved or dispersed in the culture medium coats the surfaces of the cell channels 530 and input and output channels 510, 520 to thereby reduce the flow resistance for the biological sample and the culture medium with dissolved or dispersed agents.

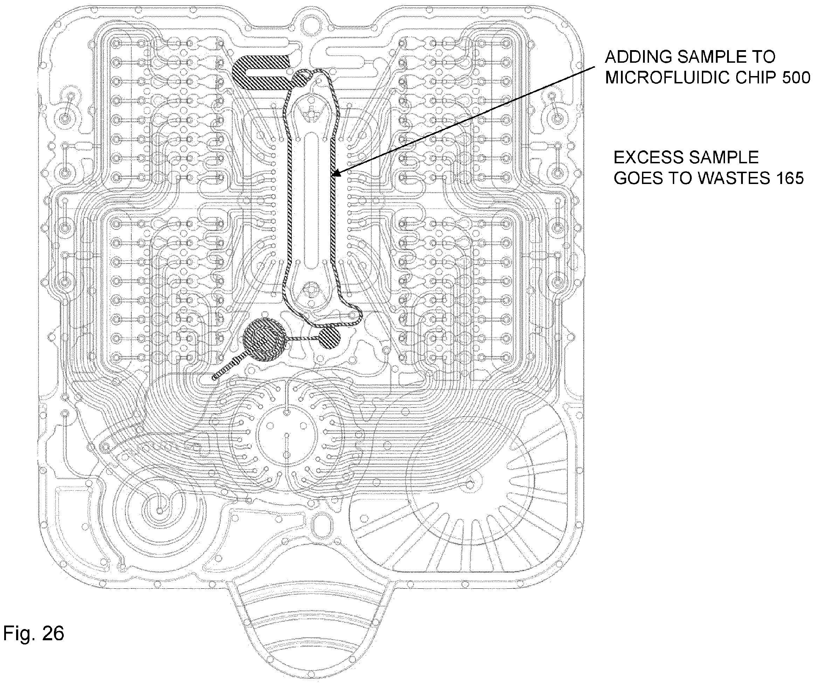

Once the wetting of the microfluidic chip 500 is finished, the filtered biological sample is pushed into microfluidic chip 500 to therein capture any cells present in the filtered biological sample in the cell channels 530, see Figs. 17, 26 and 30. The instrument thereby applies a pressure at the pressure port 108F in fluid connection with the sample reservoir 160 via the sample waste chamber 165 and the passage 164. The filtered biological sample in the sample reservoir 160 is thereby pushed through the entrance hole 154 into the T-shaped channel 153 and further into the holes 554 and second channel 553 in the second column 550B in the chip carrier 550. The filtered biological sample is further pushed into the input channels 510 via the second input ports 514 and then into the cell channels 530. The filtered biological sample is further flown out through the second ends 534 of the cell channels 530, into the output channels 520 and out through the output ports 522 and then leaves the microfluidic chip 500.

Cells present in the filtered biological sample will be trapped in the cell channels 530 by the cell blocks 535 provided therein. Excess filtered biological sample is allowed to flow into the holes 552 and first channel 511 in the first column 550A and further into the back channel reservoir 130 and the back channel waste chamber 136. During the filling process, excess filtered biological sample may also leave the input channels 510 through the first input ports 512 and then into the holes 556 and third channel 555 in the third column 550C to thereby enter the waste chambers 190.

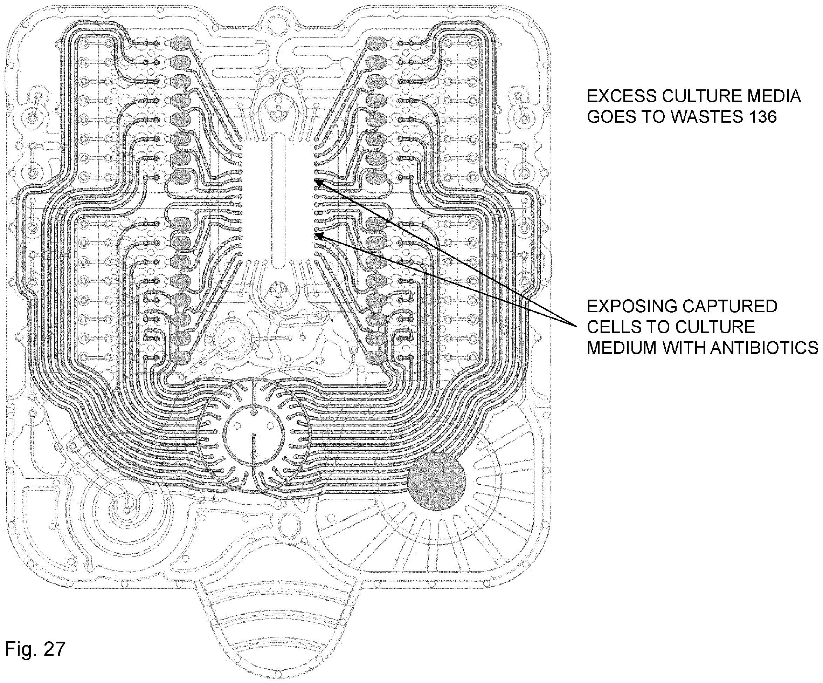

At this point, cells present in the biological sample have been captured in the cell channels 530 in the microfluidic chip 500 and can now be exposed to one or more agents dissolved or dispersed in the culture medium in the loaded medium reservoirs 170, see Figs. 17 and 27. Accordingly, the instrument applies pressure at the pressure ports 108B, 108D that are in fluid connection with the medium reservoirs 170 via the channel 176 and the membrane chambers 106. The applied pressure presses the culture medium with dissolved or dispersed agent, dissolved control chemical or no dissolved chemicals or agent, out through the drainage hole 177 and the channel 178 at the bottom surface 103 of the substrate 100 and into the respective holes 558 in the fourth column 550D of the chip carrier 550, see Fig. 31. The culture medium with dissolved or dispersed agent or chemical is further pushed into the first input ports 512 of the input channels 520 and flows into the cell channels 530 and out into the output channels 520 and the output ports 522. This flow of culture medium means that cells captured in cell channels 530 in one set 531 of cell channels 530 is exposed to a defined concentration of an agent, whereas cells captured in cell channels 530 in another set 531 of cell channels 530 may be exposed to another defined concentration of the agent, to a defined concentration of another agent, to a control chemical or merely to culture medium. By having multiple sets 531 of cell channels 530, such as 2×*N*, or 32, as shown in the figures, multiple different agents and various concentrations of these multiple different agents can be tested for one particular biological sample and still allowing internal controls in one or more sets 531 of cell channels 530. Excess culture medium with any dissolved or dispersed agent or control chemical is flown from the output ports 522 into the holes 552 and first channel 551 of the first column 550A and then out through the back channel reservoir 130 and the back channel waste chamber 136. Excess culture medium may also leave the input channel 510 through the second input port 514 and out into holes 554 and third channels 555 of the third column 550C and then out into the waste chambers 190.

The response of the cells trapped in the cell channels 530 to the various agents can then be monitored and analyzed by the instrument. In a particular embodiment, the phenotypic response of the cells to the various agents is monitored and analyzed by the instrument. Various types of phenotypic responses and phenotype characteristics could be monitored and analyzed including, but not limited to, growth rate, shape, size, form of growth rate curve defining growth rate over time, form of length curve defining cell length over time, form of area curve defining cell area over time, color, optical density, electrical conductivity, heat production, surface antigen composition as observed by affinity reagents, absorption spectra, and a mixture of at least two such phenotype characteristics.

Growth rate is a phenotypic characteristic or trait that can advantageously be used to determine the response of the captured cells to the various agents. Growth rate can be determined, for instance, by monitoring the number of cells in each cell channel 530 as the number will increase over time for growing cells. Alternatively, or in addition, grow rate can be determined by monitoring the length of the portion of a cell channel 530 occupied by cells. This length will increase over time for growing cells but remain the same for non-viable and non-growing cells. Alternatively, or in addition, grow rate can be determined by monitoring the area or length of cells segmented in images of the cell channels 530

The growth rate over time typically may vary depending on the presence of any agent in the culture medium and/or the concentration of the agent. In some instances, the cells grow exponentially, whereas in other instances the cells grow in more periodic ways. Accordingly, the shape or form of the growth rate curve can be used to determine the phenotypic response of the cells to the various agents.

Other phenotypic characteristics that may vary for the cells in the absence versus presence of the agents include the shape, size, color and optical density. Optical density, color or other spectral properties may differ depending on contents of the cells, shape of the cells, etc. Thus, optical properties can be used to determine the responses of the cells to the various agents. Conductivity and heat production will depend on the chemical composition and metabolic state of the cells and can therefore constitute the basis for determining the cellular responses to the agents.

The instrument is preferably configured to monitor the cells trapped in the cell channels 530 either more or less continuously or at multiple time instances as the culture medium with dissolved or dispersed agents are flowing through the cell channels 530. For instance, the instrument could include one or more video cameras for monitoring the response of the cells to the various agents or one or more cameras taking pictures of the sets 531 of cell channels at selected time instances.

In a particular embodiment, the instrument takes pictures of the cells in the cell channels 530 using a microscopy, such as a phase contrast microscope, connected to a camera, such as charge-coupled device (CCD) and complementary metal-oxide semiconductor (CMOS) camera, or a confocal scanning system for fluorescence, Raman imaging, Coherent Anti-stokes Raman Scattering (CARS), Stimulated Raman Scattering (SRS) and similar chemically sensitive techniques that gives spectral changes for dead and live cells. This includes measurements in one or several wavelengths with or without contrast enhancing additions to the growth medium, such as chemically specific probes and dyes.

Conductivity and/or heat production could be measured by electrodes or sensors of the instrument arranged in or in connection with the cell channels.

In a particular embodiment, the cartridge 1 and the instrument is used for AST of bacteria present in a biological sample, such as a urine sample. A substrate 100 designed as shown in the figures could then, for instance, be preloaded with five different antibiotics, each in five different concentrations in 25 of the 32 medium reservoirs 170. The remaining 7 medium reservoirs 170 may either be empty, i.e., not preloaded with any chemicals, or at least a portion thereof could be preloaded with one or more control chemicals. The phenotypic response, such as in terms of growth rates, of bacteria in the biological sample, such as urine sample, can then be determined using the cartridge 1 and the instrument in a very short period of time, typically within one or a few hours, or even less than one hour. This should be compared to the traditional culture-based AST methods requiring culturing of bacteria at least overnight.

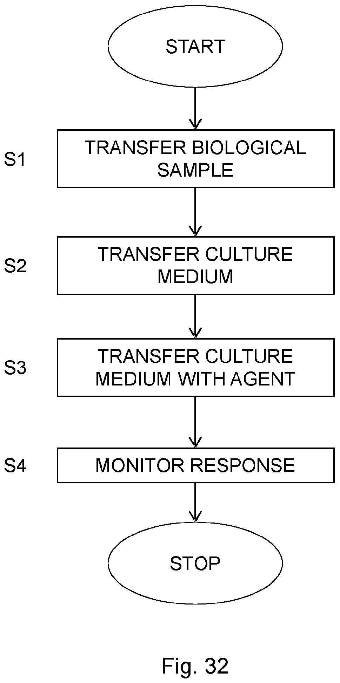

Another aspect of the invention relates to a method of analyzing a biological sample, see Fig. 32. The method comprises transferring, in step S1, a biological sample comprising cells to a plurality of sets 531 of cell channels 530 in a microfluidic chip 500. The method also comprises transferring, in step S2, a culture medium to a plurality of medium reservoirs 170. Each medium reservoir 170 of the plurality of medium reservoirs 170 is configured to be in fluid connection with a respective set 531 of cell channels 530 of the plurality of sets 531 of cell channels 530. At least one of the medium reservoirs 170 of the plurality of medium reservoirs 170 is preloaded with a defined amount of an agent configured to be dissolved or dispersed in the culture medium. The method further comprises transferring, in step S3, culture medium with the dissolved or dispersed agent from each medium reservoir 170, in which the agent is preloaded, to the respective set 531 of cell channels 530. The method additionally comprises monitoring, in step S4, a response of the cells to the agent in the respective sets 531 of cell channels 530.

In an embodiment, step S3 comprises transferring the culture medium with the dissolved or dispersed agent or the culture medium without any agent from each medium reservoir 170 of the plurality 170 of medium reservoirs to the respective set 531 of cell channels 530.

In an embodiment, at least some of the plurality of medium reservoirs 170 are preloaded with different amounts of the agent or different agents configured to be dissolved or dispersed in the culture medium.

In an embodiment, step S1 comprises transferring the biological sample comprising cells to a plurality of sets 531 of cell channels 530 in a microfluidic chip 500 arranged in a chip chamber 105 of a cartridge 1 according to any of the embodiments as disclosed herein. In this embodiment, step S2 comprises transferring the culture medium to a plurality of medium reservoirs 170 of the cartridge 1 according to any of the embodiments as disclosed herein.

Herein, various additional aspects of the invention will be described in more detail. Any of these other additional aspects can be combined with each other and/or with the previously described aspect of the invention.

A first additional aspect relates to a cartridge 1 comprising a substrate 100. The substrate 100 comprises a blister chamber 110 configured to house a medium blister 400 comprising culture medium. The substrate 100 also comprises a pressure port 108E configured to be in fluid connection with a pressure source configured to apply pressurized fluid, preferably pressurized gas, and more preferably pressurized air, at the pressure port 108E. The substrate 100 further comprises a pressure channel 116 interconnecting the pressure port 108E and the blister chamber 110. The cartridge 1 also comprises a top lid 200 attached to an upper surface 102 of the substrate 100 and configured to enclose the medium blister 400 in the blister chamber 110. According to this first additional aspect, the blister chamber 110 comprises a central recess 111 and a peripheral chamber bottom 112. The medium blister 400 is attached to the peripheral chamber bottom 112. The central recess 111 comprises a drainage hole 113 and at least one cutter 114 and has a depth sufficiently deep to space the at least one cutter 114 a distance from a bottom surface of the medium blister 400. The at least one cutter 114 is configured to penetrate the bottom surface of the medium blister 400 upon application of the pressurized fluid, preferably pressurized gas, and more preferably pressurized air, at the pressure port 108E and flowing into the blister chamber 110 through the pressure channel 116 and in between the top lid 200 and an upper surface of the medium blister 400, thereby pushing the medium blister 400 downwards towards the central recess 111, opening the bottom surface of the medium blister 400 and pushing culture medium in the medium blister 400 through the drainage hole 113.

In an embodiment, the cutter 114 comprises a drainage channel 118 in a side 117 of the cutter 114 facing the drainage hole 113. In this embodiment, the drainage channel 118 is configured to direct culture medium from the medium blister 400 into the drainage hole.

In an embodiment, a side 117 of the cutter facing the drainage hole 113 is arced around a portion of the drainage hole 113.

A second additional aspect relates to a cartridge 1 comprising a substrate 100. The substrate 100 comprises a medium valve chamber 120 comprising, in a bottom surface of the medium valve chamber 120, an entrance hole 121 in fluid connection with a culture medium source 110, 400, a back channel hole 122 in fluid connection with a back channel reservoir 130 and a plurality of medium reservoir holes 123 each in fluid connection with a respective medium reservoir 170. The substrate 100 also comprises a culture medium valve 420 attached to a peripheral portion 124 of the bottom surface of the medium valve chamber 120. The substrate 100 further comprises a pressure port 108G configured to be in fluid connection with a pressure source configured to apply pressurized fluid, preferably pressurized gas, and more preferably pressurized air, at the pressure port 108G, and a pressure channel 129A interconnecting the pressure port 108G and the medium valve chamber 120. The culture medium valve 420 is configured, upon application of a first fluid pressure, preferably gas pressure and more preferably air pressure, at the pressure port 108G, to redirect an inflow of culture medium from the entrance hole 121 into the back channel hole 122 and the plurality of medium reservoir holes 123 and towards the back channel reservoir 130 and the medium reservoirs 170. The culture medium valve 420 is also configured, upon application of a second fluid pressure, preferably gas pressure and more preferably air pressure at the pressure port 108G that is higher than the first fluid pressure, to press against the bottom surface of the medium valve chamber 120 and close the entrance hole 121, the back channel hole 122 and the plurality of medium reservoir holes 123 to prevent any culture medium flow between the medium reservoirs 170 and the back channel reservoir 130.

In an embodiment, the medium valve chamber 120 comprises, in the bottom surface of the medium valve chamber 120, a central entrance hole 121, the back channel hole and the plurality of medium reservoir holes 123 circumferentially arranged around the central entrance hole 121.

In an embodiment, the culture medium valve 420 is a disc made of a flexible material, preferably a thermoplastic elastomer.

In an embodiment, the pressure port 108E is a first pressure port 108. In this embodiment, the substrate 100 further comprises a second pressure port 108A in fluid connection with the back channel reservoir 130. The culture medium valve 420 is configured, upon application of the first fluid pressure, preferably gas pressure and more preferably air pressure, at the first pressure port 108G and upon application of a fluid pressure, preferably gas pressure and more preferably air pressure, at the second pressure port 108A, to initially redirect the inflow of culture medium from the entrance hole 121 into the plurality of medium reservoir holes 123 towards the medium reservoirs 170 and to sequentially, when the medium reservoirs 170 are filled with culture medium, redirect the inflow of culture medium from the entrance hole 121 into the back channel hole 122 towards the back channel reservoir 130.

In an embodiment, the culture medium valve 420 is configured, upon application of the first fluid pressure, preferably gas pressure and more preferably air pressure, at the first pressure port 108G and upon application of the fluid pressure, preferably gas pressure and more preferably air pressure, at the second pressure port 108A, to initially redirect the inflow of culture medium from the entrance hole 121 into the plurality of medium reservoir holes 123 towards the medium reservoirs 170 and to sequentially, upon release of the fluid pressure, preferably gas pressure and more preferably air pressure, at the second pressure port 108A, redirect the inflow of culture medium from the entrance hole 121 into the back channel hole 122 towards the back channel reservoir 130.

A third additional aspect relates to a cartridge 1 comprising a substrate 100. The substrate comprises a plurality of medium reservoirs 170. At least a portion of the plurality of the medium reservoirs 170 comprises different amounts of an agent and/or different agents. The cartridge 1 also comprises a microfluidic chip 500 comprising a plurality of sets 531 of cell channels 530 configured to capture cells from a biological sample. Each set 531 of cell channels 530 is in fluid connection with a respective medium reservoir 170 of the plurality of medium reservoirs 170. The cartridge 1 further comprises a culture medium source 110, 400 in fluid connection with the plurality of medium reservoirs 170. Each medium reservoir 170 comprises an entrance hole 171A, 171B in fluid connection with the culture medium source 110, 400 and a pillar chamber 172A, 172B comprising the entrance hole 171A, 171B. Each medium reservoir 170 also comprises a chamber part 174A, 174B interconnected with the entrance pillar chamber 172A, 172B though a waist 173A, 173B configured to exert a flow resistance to culture medium and a drainage hole 177 in fluid connection with a respect set 531 of cell channels 530 of the plurality of sets 531 of cell channels 530. In this aspect, culture medium entering the medium reservoirs 170 through the entrance holes 171A, 171B is, due to the flow resistance exerted by the waists 173A, 173B, configured to initially fill the pillar chambers 172A, 172B venting air present in the pillar chambers 172A, 172B out through the waists 173A, 173B and sequentially, when the pillar chambers 172A, 172B are filled with culture medium, fill the chamber parts 174A, 174B thereby filling the medium reservoirs 170 with a respective predefined volume of culture medium and obtaining a respective predefined concentration of the agent or different agents.

In an embodiment, each medium reservoir 170 of the plurality of medium reservoirs 170 comprises back chamber 170A and a front chamber 170B. The back chamber 170A comprises an entrance hole 171A in fluid connection with the culture medium source 110, 400. The back chamber 170A also comprises a pillar chamber 172A comprising the entrance hole 171A and a chamber part 174A interconnected with the entrance pillar chamber 172A though a waist 173A configured to exert a flow resistance to culture medium and in fluid connection with a membrane chamber 106. The front chamber 170B comprises an entrance hole 171B in fluid connection with the culture medium source 110, 400 and a pillar chamber 172B comprising the entrance hole 171B. The front chamber 170B also comprises a chamber part 174B interconnected with the entrance pillar chamber 172B though a waist 173B configured to exert a flow resistance to culture medium and a drainage hole 177 in fluid connection with a respect set 531 of cell channels 530 of the plurality of sets 531 of cell channels 530.

In an embodiment, the front chambers 170B exert a higher flow resistance to the culture medium than the back chambers 170A to direct culture medium into the entrance holes 171A of the back chambers 170A.

In an embodiment, the membrane chamber 106 is in fluid connection with a pressure port 108B, 108D configured to be in fluid connection with a pressure source configured to apply pressurized fluid, preferably pressurized gas, and more preferably pressurized air, at the pressure port 108B, 108D to press culture medium in the back chambers 170A into a respective front chamber 170B.

In an embodiment, the front chambers 170B comprise a respective step 273 arranged between the drainage holes 177 and the chamber parts 174B, wherein the steps 273 are configured to induce a turbulence in culture medium during filling of the chamber parts 174B to promote mixing of the agent or different agents and the culture medium.

A fourth additional aspect relates to a cartridge 1 comprising a substrate 100. The substrate 100 comprises a sample chamber 140 configured to comprise a biological sample comprising cells and comprising a drainage hole 141. The substrate 100 also comprises a dome pump chamber 180 having a bottom surface comprising a drainage hole 182 in fluid connection with the sample chamber 140. The cartridge 1 also comprises a dome shaped pump 410 attached to a peripheral part 181 of the bottom surface of the dome pump chamber 180 and configured to enclose a defined volume of air. The substrate 100 further comprises a pressure port 108E in fluid connection with the dome pump chamber 180 and configured to be in fluid connection with a pressure source configured to apply pressurized fluid, preferably pressurized gas, and more preferably pressurized air, at the pressure port 108E to press the dome shaped pump 410 downwards towards the bottom surface of the dome pump chamber 180 to press a defined volume of air contained by the dome shaped pump 410 in the dome pump chamber 180 through the drainage hole 182 of the dome pump chamber 180 and into the sample chamber 140 to push a defined volume of the biological sample into the drainage hole 141 of the sample chamber 140.

In an embodiment, the dome shaped pump 410 is a flexible dome, preferably a thermoplastic elastomer dome.

In an embodiment, the substrate 100 further comprises a filter chamber 146 comprising a sample filter 630 in fluid connection with the drainage hole 141 of the sample chamber 140.

In an embodiment, the substrate 100 also comprises a sample valve chamber 150 having a bottom surface comprising an entrance hole 148 in fluid connection with the filter chamber 146 and a drainage hole 151. The substrate 100 further comprises a pressure port 108G configured to be in fluid connection with a pressure source configured to apply pressurized fluid, preferably pressurized gas, and more preferably pressurized air, at the pressure port 108G, and a pressure channel 129A, 155 interconnecting the pressure port 108G and the sample valve chamber 150. The cartridge 1 further comprises a sample valve 430 attached to a peripheral portion of the bottom surface of the sample valve chamber 150. In this embodiment, the sample valve 430 is configured, upon application of a fluid pressure, preferably gas pressure and more preferably air pressure, at the pressure port 108G, to press against the bottom surface of the sample valve chamber 150 and close the entrance hole 148 and the drainage hole 151 to prevent any back flow of biological sample into the sample valve chamber 150.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A cartridge (1) comprising:
a chip chamber (105) configured to house a microfluidic chip (500) comprising a plurality of sets (531) of cell channels (530) configured to capture cells from a biological sample;
a sample chamber (140) configured to receive the biological sample and be in fluid connection with the plurality of sets (531) of cell channels (530);
a plurality of medium reservoirs (170), wherein each medium reservoir (170) of the plurality of medium reservoirs (170) is configured to be in fluid connection with a respective set (531) of cell channels (530) of the plurality of sets (531) of cell channels (530); and
a culture medium source (110, 400) in fluid connection with the plurality of medium reservoirs (170) and configured to supply a culture medium to the plurality of medium reservoirs (170).

2. The cartridge of claim 1, wherein the plurality of medium reservoirs (170) has a substantially same shape and/or internal volume.

3. The cartridge of claim 1 or 2, wherein at least some of the plurality of medium reservoirs (170) are preloaded with different amounts of an agent or different agents configured to be dissolved or dispersed in the culture medium.

4. The cartridge of claim 3, wherein a first set of the plurality of medium reservoirs (170) is preloaded with the different amounts of an agent or the different agents, and a second set of the plurality of medium reservoirs (170) is not preloaded with any agent.

5. The cartridge of claim 3 or 4, wherein the agent is an antimicrobial agent or the different agents are different antimicrobial agents.

6. The cartridge of any one of claims 1 to 5, further comprising:
a medium valve chamber (120) comprising:
an entrance hole (121) in fluid communication with the culture medium source (110, 400); and
a plurality of medium reservoir holes (123), wherein each medium reservoir hole (123) of the plurality of medium reservoir holes (123) is in fluid communication with a respective medium reservoir (170) of the plurality of medium reservoirs (170); and
a culture medium valve (420) configured to direct culture medium supplied through the entrance hole (121) from the culture medium source (110, 400) into the plurality of medium reservoir holes (123).

7. The cartridge of claim 6, wherein the culture medium source (110, 400) comprises:
a culture medium container (400) prepackaged with the culture medium; and
a chamber (110) configured to house the culture medium container (400) and comprising:
a drainage hole (113) in fluid communication with the plurality of medium reservoirs (170); and
a cutter (114) configured to cut a surface of the culture medium container (400) to supply the culture medium into the drainage hole (113).

8. The cartridge of any one of claims 1 to 7, further comprising a pump (410) configured to move biological sample from the sample chamber (140) into the plurality of sets (531) of cell channels (530).

9. The cartridge of any one of claims 1 to 8, further comprising a surfactant chamber (128) configured to be in fluid communication with the plurality of sets (531) of cell channels (530) and the culture medium source (110, 400), wherein
the surfactant chamber (128) is preloaded with a surfactant configured to be dissolved or dispersed in culture medium supplied from the culture medium source (110, 400); and
the surfactant dissolved or dispersed in the culture medium is configured to be supplied to the plurality of sets (531) of cell channels (530).

10. The cartridge of any one of claims 1 to 9, wherein the chip chamber (105) comprises the microfluidic chip (500) comprising the plurality of sets (531) of cell channels (530) configured to capture cells from the biological sample.

11. The cartridge of claim 10, further comprising:
a chip carrier (550) attached to the microfluidic chip (500) and comprising:
a first set of holes (554) configured to provide fluidic connection between the plurality of sets (531) of cell channels (530) and the sample chamber (140); and
a second set of holes (558), wherein each hole (558) of the second set of holes (558) is configured to provide fluidic connection between a respective set (531) of cell channels (530) of the plurality of sets (531) of cell channels (530) and a respective medium reservoir (170) of the plurality of medium reservoirs (170).

12. The cartridge of claim 11, wherein each set (531) of cell channels (530) of the plurality of sets (531) of cell channels (530) comprises:
a first port (514) in fluid communication with a hole (554) of the first set of holes (554); and
a second port (512) in fluid communication with a hole (558) of the second set of holes (558).

13. The cartridge of any one of claims 10 to 12, wherein each set (531) of cell channels (530) of the plurality of sets (531) of cell channels (530) is formed as a compartment in the microfluidic chip (500), which is separate from other sets (531) of cell channels (530) of the plurality of sets (531) of cell channels (530).

14. The cartridge of any one of claims 1 to 13, further comprising:
a substrate (100) comprising the chip chamber (105), the sample chamber (140), the plurality of medium reservoirs (170) and the culture medium source (110, 400); and
a lid (200) attached to the substrate (100) and comprising a window (210) configured to enable imaging the plurality of sets (531) of cell channels (530).

15. A method of analyzing a biological sample, comprising:
transferring (S1) a biological sample comprising cells to a plurality of sets (531) of cell channels (530) in a microfluidic chip (500) in the cartridge of claim 10;
transferring (S2) a culture medium to a plurality of medium reservoirs (170), wherein each medium reservoir (170) of the plurality of medium reservoirs (170) is configured to be in fluid connection with a respective set (531) of cell channels (530) of the plurality of sets (531) of cell channels (530) in the microfluidic chip (500), wherein at least one of the medium reservoirs (170) of the plurality of medium reservoirs (170) is preloaded with a defined amount of an agent configured to be dissolved or dispersed in the culture medium;
transferring (S3) culture medium with the dissolved or dispersed agent from each medium reservoir (170), in which the agent is preloaded, to the respective set (531) of cell channels (530) in the microfluidic chip (500); and
monitoring (S4) a response of the cells to the agent in the respective sets (531) of cell channels (530) in the microfluidic chip (500).

16. The method according to claim 15, wherein
transferring (S3) the culture medium with the dissolved or dispersed agent comprises transferring (S3) the culture medium with the dissolved or dispersed agent or the culture medium without any agent from each medium reservoir (170) of the plurality of medium reservoirs (170) to the respective set (531) of cell channels (530) in the microfluidic chip (500).

17. The method of claim 15 or 16, wherein
at least some of the plurality of medium reservoirs (170) are preloaded with different amounts of the agent or different agents configured to be dissolved or dispersed in the culture medium.

18. The method of any of the claims 15 to 17, wherein
transferring (S1) the biological sample comprises transferring (S1) the biological sample comprising cells to a plurality of sets (531) of cell channels (530) in the microfluidic chip (500) arranged in a chip chamber (105) of a cartridge (1) of any of the claims 1 to 14; and
transferring (S2) the culture medium comprises transferring (S2) the culture medium to a plurality of medium reservoirs (170) of the cartridge (1) of any of the claims 1 to 14.

## Patentansprüche

1. Kartusche (1), umfassend:
eine Chipkammer (105), die zum Unterbringen eines mikrofluidischen Chips (500) konfiguriert ist, der eine Vielzahl von Sätzen (531) von Zellkanälen (530) umfasst, die dazu konfiguriert ist, Zellen aus einer biologischen Probe aufzufangen;
eine Probenkammer (140), die dazu konfiguriert ist, die biologische Probe aufzunehmen und in Fluidverbindung mit der Vielzahl von Sätzen (531) von Zellkanälen (530) zu stehen;
eine Vielzahl von Medienbehältern (170), wobei jeder Medienbehälter (170) der Vielzahl von Medienbehältern (170) dazu konfiguriert ist, in Fluidverbindung mit einem jeweiligen Satz (531) von Zellkanälen (530) der Vielzahl von Sätzen (531) von Zellkanälen (530) zu stehen; und
eine Kulturmedienquelle (110, 400), die in Fluidverbindung mit der Vielzahl von Medienbehältern (170) steht und dazu konfiguriert ist, der Vielzahl von Medienbehältern (170) ein Kulturmedium zuzuführen.

2. Kartusche nach Anspruch 1, wobei die Vielzahl von Medienbehältern (170) eine im Wesentlichen gleiche Form und/oder ein im Wesentlichen gleiches Innenvolumen aufweist.

3. Kartusche nach Anspruch 1 oder 2, wobei mindestens einige der Vielzahl von Medienbehältern (170) mit unterschiedlichen Mengen eines Mittels oder unterschiedlichen Mitteln, das/die dazu konfiguriert ist/sind, in dem Kulturmittel aufgelöst oder dispergiert zu werden, vorgeladen sind.

4. Kartusche nach Anspruch 3, wobei ein erster Satz der Vielzahl von Medienbehältern (170) mit den unterschiedlichen Mengen eines Mittels oder den unterschiedlichen Mitteln vorgeladen ist und ein zweiter Satz der Vielzahl von Medienbehältern (170) mit keinem Mittel vorgeladen ist.

5. Kartusche nach Anspruch 3 oder 4, wobei das Mittel ein antimikrobielles Mittel ist oder die unterschiedlichen Mittel unterschiedliche antimikrobielle Mittel sind.

6. Kartusche nach einem der Ansprüche 1 bis 5, ferner umfassend:
eine Medienventilkammer (120), umfassend:
ein Eingangsloch (121), das in Fluidkommunikation mit der Kulturmedienquelle (110, 400) steht; und
eine Vielzahl von Medienbehälterlöchern (123), wobei jedes Medienbehälterloch (123) der Vielzahl von Medienbehälterlöchern (123) in Fluidverbindung mit einem jeweiligen Medienbehälter (170) der Vielzahl von Medienbehältern (170) steht; und
ein Kulturmedienventil (420), das dazu konfiguriert ist, durch das Eingangsloch (123) zugeführtes Kulturmedium aus der Kulturmedienquelle (110, 400) in die Vielzahl von Medienbehälterlöchern (123) zu leiten.

7. Kartusche nach Anspruch 6, wobei die Kulturmedienquelle (110, 400) Folgendes umfasst:
einen Kulturmedienbehälter (400), der mit dem Kulturmedium vorverpackt ist; und
eine Kammer (110), die zum Unterbringen des Kulturmedienbehälters (400) konfiguriert ist und Folgendes umfasst:
ein Ablaufloch (113), das in Fluidverbindung mit der Vielzahl von Medienbehältern (170) steht; und
eine Schneidvorrichtung (114), die dazu konfiguriert ist, eine Fläche des Kulturmedienbehälters (400) zu schneiden, um das Kulturmedium in das Ablaufloch (113) zuzuführen.

8. Kartusche nach einem der Ansprüche 1 bis 7, ferner umfassend eine Pumpe (410), die dazu konfiguriert ist, eine biologische Probe aus der Probenkammer (140) in die Vielzahl von Sätzen (531) von Zellkanälen (530) zu bewegen.

9. Kartusche nach einem der Ansprüche 1 bis 8, ferner umfassend eine Tensidkammer (128), die dazu konfiguriert ist, in Fluidverbindung mit der Vielzahl von Sätzen (531) von Zellkanälen (530) und der Kulturmedienquelle (110, 400) zu stehen, wobei
die Tensidkammer (128) mit einem Tensid vorgeladen ist, das dazu konfiguriert ist, in aus der Kulturmedienquelle (110, 400) zugeführtem Kulturmedium aufgelöst oder dispergiert zu werden; und
das in dem Kulturmedium aufgelöste oder dispergierte Tensid dazu konfiguriert ist, der Vielzahl von Sätzen (531) von Zellkanälen (530) zugeführt zu werden.

10. Kartusche nach einem der Ansprüche 1 bis 9, wobei die Chipkammer (105) den mikrofluidischen Chip (500) umfasst, der die Vielzahl von Sätzen (531) von Zellkanälen (530) umfasst, die dazu konfiguriert ist, Zellen aus der biologischen Probe aufzufangen.

11. Kartusche nach Anspruch 10, ferner umfassend:
einen Chipträger (550), der an dem mikrofluidischen Chip (500) angebracht ist und Folgendes umfasst:
einen ersten Satz von Löchern (554), der dazu konfiguriert ist, eine fluidische Verbindung zwischen der Vielzahl von Sätzen (531) von Zellkanälen (530) und der Probenkammer (140) bereitzustellen; und
einen zweiten Satz von Löchern (558), wobei jedes Loch (558) des zweiten Satzes von Löchern (558) dazu konfiguriert ist, eine fluidische Verbindung zwischen einem jeweiligen Satz (531) von Zellkanälen (530) der Vielzahl von Sätzen (531) von Zellkanälen (530) und einem jeweiligen Medienbehälter (170) der Vielzahl von Medienbehältern (170) bereitzustellen.

12. Kartusche nach Anspruch 11, wobei jeder Satz (531) von Zellkanälen (530) der Vielzahl von Sätzen (531) von Zellkanälen (530) Folgendes umfasst:
einen ersten Anschluss (514), der in Fluidverbindung mit einem Loch (554) des ersten Satzes von Löchern (554) steht; und
einen zweiten Anschluss (512), der in Fluidverbindung mit einem Loch (558) des zweiten Satzes von Löchern (558) steht.

13. Kartusche nach einem der Ansprüche 10 bis 12, wobei jeder Satz (531) von Zellkanälen (530) der Vielzahl von Sätzen (531) von Zellkanälen (530) als ein Fach in dem mikrofluidischen Chip (500) ausgebildet ist, das von anderen Sätzen (531) von Zellkanälen (530) der Vielzahl von Sätzen (531) von Zellkanälen (530) getrennt ist.

14. Kartusche nach einem der Ansprüche 1 bis 13, ferner umfassend:
ein Substrat (100), das die Chipkammer (105), die Probenkammer (140), die Vielzahl von Medienbehältern (170) und die Kulturmedienquelle (110, 400) umfasst; und
einen Deckel (200), der an dem Substrat (100) angebracht ist und ein Fenster (210) umfasst, das dazu konfiguriert ist, eine Bildgebung der Vielzahl von Sätzen (531) von Zellkanälen (530) zu ermöglichen.

15. Verfahren zur Analyse einer biologischen Probe, umfassend:
Transferieren (S1) einer biologischen Probe, die Zellen umfasst, in eine Vielzahl von Sätzen (531) von Zellkanälen (530) in einem mikrofluidischen Chip (500) in der Kartusche nach Anspruch 10;
Transferieren (S2) eines Kulturmediums in eine Vielzahl von Medienbehältern (170), wobei jeder Medienbehälter (170) der Vielzahl von Medienbehältern (170) dazu konfiguriert ist, in Fluidverbindung mit einem jeweiligen Satz (531) von Zellkanälen (530) der Vielzahl von Sätzen (531) von Zellkanälen (530) in dem mikrofluidischen Chip (500) zu stehen, wobei mindestens einer der Medienbehälter (170) der Vielzahl von Medienbehältern (170) mit einer definierten Menge eines Mittels vorgeladen ist, das dazu konfiguriert ist, in dem Kulturmedium aufgelöst oder dispergiert zu werden;
Transferieren (S3) von Kulturmedium mit dem aufgelösten oder dispergierten Mittel aus jedem Medienbehälter (170), in den das Mittel vorgeladen ist, in den jeweiligen Satz (531) von Zellkanälen (530) in dem mikrofluidischen Chip (500); und
Überwachen (S4) einer Reaktion der Zellen auf das Mittel in den jeweiligen Sätzen (531) von Zellkanälen (530) in dem mikrofluidischen Chip (500).

16. Verfahren nach Anspruch 15, wobei
das Transferieren (S3) des Kulturmediums mit dem aufgelösten oder dispergierten Mittel Transferieren (S3) des Kulturmediums mit dem aufgelösten oder dispergierten Mittel oder des Kulturmediums ohne irgendein Mittel aus jedem Medienbehälter (170) der Vielzahl von Medienbehältern (170) in den jeweiligen Satz (531) von Zellkanälen (530) in dem mikrofluidischen Chip (500) umfasst.

17. Verfahren nach Anspruch 15 oder 16, wobei
mindestens einige der Vielzahl von Medienbehältern (170) mit unterschiedlichen Mengen des Mittels oder mit unterschiedlichen Mitteln, das/die dazu konfiguriert ist/sind, in dem Kulturmedium aufgelöst oder dispergiert zu werden, vorgeladen sind.

18. Verfahren nach einem der Ansprüche 15 bis 17, wobei
das Transferieren (S1) der biologischen Probe Transferieren (S1) der biologischen Probe, die Zellen umfasst, in eine Vielzahl von Sätzen (531) von Zellkanälen (530) in dem mikrofluidischen Chip (500), der in einer Chipkammer (105) einer Kartusche (1) nach einem der Ansprüche 1 bis 14 angeordnet ist, umfasst; und
das Transferieren (S2) des Kulturmediums Transferieren (S2) des Kulturmediums in eine Vielzahl von Medienbehältern (170) der Kartusche (1) nach einem der Ansprüche 1 bis 14 umfasst.

## Revendications

1. Cartouche (1) comprenant :
une chambre à puce (105) conçue pour loger une puce microfluidique (500) comprenant une pluralité d'ensembles (531) de canaux cellulaires (530) conçus pour capturer des cellules à partir d'un échantillon biologique ;
une chambre à échantillon (140) conçue pour recevoir l'échantillon biologique et être en liaison fluidique avec la pluralité d'ensembles (531) de canaux cellulaires (530) ;
une pluralité de réservoirs de milieu (170), chaque réservoir de milieu (170) de la pluralité de réservoirs de milieu (170) étant conçu pour être en liaison fluidique avec un ensemble (531) respectif de canaux cellulaires (530) de la pluralité d'ensembles (531) de canaux cellulaires (530) ; et
une source de milieu de culture (110, 400) en liaison fluidique avec la pluralité de réservoirs de milieu (170) et conçue pour fournir un milieu de culture à la pluralité de réservoirs de milieu (170).

2. Cartouche selon la revendication 1, dans laquelle la pluralité de réservoirs de milieu (170) a sensiblement la même forme et/ou le même volume interne.

3. Cartouche selon la revendication 1 ou 2, dans laquelle au moins certains de la pluralité de réservoirs de milieu (170) sont préchargés avec différentes quantités d'un agent ou de différents agents conçus pour être dissous ou dispersés dans le milieu de culture.

4. Cartouche selon la revendication 3, dans laquelle un premier ensemble de la pluralité de réservoirs de milieu (170) est préchargé avec les différentes quantités d'un agent ou des différents agents, et un second ensemble de la pluralité de réservoirs de milieu (170) n'est pas préchargé avec n'importe quel agent.

5. Cartouche selon la revendication 3 ou 4, dans laquelle l'agent est un agent antimicrobien ou les différents agents sont des agents antimicrobiens différents.

6. Cartouche selon l'une quelconque des revendications 1 à 5, comprenant en outre :
une chambre de soupape de milieu (120) comprenant :
un trou d'entrée (121) en communication fluidique avec la source de milieu de culture (110, 400) ; et
une pluralité de trous de réservoir de milieu (123), chaque trou de réservoir de milieu (123) de la pluralité de trous de réservoir de milieu (123) étant en communication fluidique avec un réservoir de milieu (170) respectif de la pluralité de réservoirs de milieu (170) ; et
une soupape de milieu de culture (420) conçue pour diriger un milieu de culture fourni à travers le trou d'entrée (121) de la source de milieu de culture (110, 400) dans la pluralité de trous de réservoir de milieu (123).

7. Cartouche selon la revendication 6, dans laquelle la source de milieu de culture (110, 400) comprend :
un récipient de milieu de culture (400) préemballé avec le milieu de culture ; et
une chambre (110) conçue pour loger le récipient de milieu de culture (400) et comprenant :
un trou de drainage (113) en communication fluidique avec la pluralité de réservoirs de milieu (170) ; et
un dispositif de coupe (114) conçu pour couper une surface du récipient de milieu de culture (400) afin d'amener le milieu de culture dans le trou de drainage (113).

8. Cartouche selon l'une quelconque des revendications 1 à 7, comprenant en outre une pompe (410) conçue pour déplacer l'échantillon biologique de la chambre à échantillon (140) dans la pluralité d'ensembles (531) de canaux cellulaires (530).

9. Cartouche selon l'une quelconque des revendications 1 à 8, comprenant en outre une chambre de tensioactif (128) conçue pour être en communication fluidique avec la pluralité d'ensembles (531) de canaux cellulaires (530) et la source de milieu de culture (110, 400), dans laquelle
la chambre de tensioactif (128) est préchargée avec un tensioactif conçu pour être dissous ou dispersé dans un milieu de culture fourni par la source de milieu de culture (110, 400) ; et
le tensioactif dissous ou dispersé dans le milieu de culture est conçu pour être fourni à la pluralité d'ensembles (531) de canaux cellulaires (530).

10. Cartouche selon l'une quelconque des revendications 1 à 9, dans laquelle la chambre à puce (105) comprend la puce microfluidique (500) comprenant la pluralité d'ensembles (531) de canaux cellulaires (530) conçus pour capturer des cellules de l'échantillon biologique.

11. Cartouche selon la revendication 10, comprenant en outre :
un support de puce (550) fixé à la puce microfluidique (500) et comprenant :
un premier ensemble de trous (554) conçu pour assurer une liaison fluidique entre la pluralité d'ensembles (531) de canaux cellulaires (530) et la chambre à échantillon (140) ; et
un second ensemble de trous (558), chaque trou (558) du second ensemble de trous (558) étant conçu pour assurer une liaison fluidique entre un ensemble (531) respectif de canaux cellulaires (530) de la pluralité d'ensembles (531) de canaux cellulaires (530) et un réservoir de milieu (170) respectif de la pluralité de réservoirs de milieu (170).

12. Cartouche selon la revendication 11, dans laquelle chaque ensemble (531) de canaux cellulaires (530) de la pluralité d'ensembles (531) de canaux cellulaires (530) comprend :
un premier orifice (514) en communication fluidique avec un trou (554) du premier ensemble de trous (554) ; et
un second orifice (512) en communication fluidique avec un trou (558) du second ensemble de trous (558).

13. Cartouche selon l'une quelconque des revendications 10 à 12, dans laquelle chaque ensemble (531) de canaux cellulaires (530) de la pluralité d'ensembles (531) de canaux cellulaires (530) est formé sous la forme d'un compartiment dans la puce microfluidique (500), qui est distinct des autres ensembles (531) de canaux cellulaires (530) de la pluralité d'ensembles (531) de canaux cellulaires (530).

14. Cartouche selon l'une quelconque des revendications 1 à 13, comprenant en outre :
un substrat (100) comprenant la chambre à puce (105), la chambre à échantillon (140), la pluralité de réservoirs de milieu (170) et la source de milieu de culture (110, 400) ; et
un couvercle (200) fixé au substrat (100) et comprenant une fenêtre (210) conçue pour permettre l'imagerie de la pluralité d'ensembles (531) de canaux cellulaires (530).

15. Procédé d'analyse d'un échantillon biologique, comprenant :
le transfert (S1) d'un échantillon biologique comprenant des cellules vers une pluralité d'ensembles (531) de canaux cellulaires (530) dans une puce microfluidique (500) dans la cartouche selon la revendication 10 ;
le transfert (S2) d'un milieu de culture vers une pluralité de réservoirs de milieu (170), dans lequel chaque réservoir de milieu (170) de la pluralité de réservoirs de milieu (170) est conçu pour être en liaison fluidique avec un ensemble (531) respectif de canaux cellulaires (530) de la pluralité d'ensembles (531) de canaux cellulaires (530) dans la puce microfluidique (500), dans lequel au moins l'un des réservoirs de milieu (170) de la pluralité de réservoirs de milieu (170) est préchargé avec une quantité définie d'un agent conçu pour être dissous ou dispersé dans le milieu de culture ;
le transfert (S3) d'un milieu de culture avec l'agent dissous ou dispersé de chaque réservoir de milieu (170), dans lequel l'agent est préchargé, vers l'ensemble (531) respectif de canaux cellulaires (530) dans la puce microfluidique (500) ; et
la surveillance (S4) d'une réponse des cellules à l'agent dans les ensembles (531) respectifs de canaux cellulaires (530) dans la puce microfluidique (500).

16. Procédé selon la revendication 15, dans lequel
le transfert (S3) du milieu de culture avec l'agent dissous ou dispersé comprend le transfert (S3) du milieu de culture avec l'agent dissous ou dispersé ou du milieu de culture sans aucun agent de chaque réservoir de milieu (170) de la pluralité de réservoirs de milieu (170) vers l'ensemble (531) respectif de canaux cellulaires (530) dans la puce microfluidique (500).

17. Procédé selon la revendication 15 ou 16, dans lequel
au moins certains de la pluralité de réservoirs de milieu (170) sont préchargés avec différentes quantités de l'agent ou des différents agents conçus pour être dissous ou dispersés dans le milieu de culture.

18. Procédé selon l'une quelconque des revendications 15 à 17, dans lequel
le transfert (S1) de l'échantillon biologique comprend le transfert (S1) de l'échantillon biologique comprenant des cellules vers une pluralité d'ensembles (531) de canaux cellulaires (530) dans la puce microfluidique (500) agencée dans une chambre à puce (105) d'une cartouche (1) selon l'une quelconque des revendications 1 à 14 ; et
le transfert (S2) du milieu de culture comprend le transfert (S2) du milieu de culture vers une pluralité de réservoirs de milieu (170) de la cartouche (1) selon l'une quelconque des revendications 1 à 14.
